(19) Europäisches Patentamt
European Patent Office
Office européen des brevets

(11) Numéro de publication : 0 390 683 B1

(12) # FASCICULE DE BREVET EUROPEEN

(45) Date de publication du fascicule du brevet : **27.10.93 Bulletin 93/43**

(21) Numéro de dépôt : **90400848.9**

(22) Date de dépôt : **28.03.90**

(51) Int. Cl.$^5$ : **C07C 49/747,** C07C 49/753, C07D 307/32, A61K 31/125, A61K 31/34, A61K 7/40, C07C 45/74, C07C 45/62, C07C 45/68

(54) **Nouveaux dérivés de benzyl-cyclanones, leur procédé de préparation et compositions cosmétiques et pharmaceutiques les contenant.**

(30) Priorité : **31.03.89 FR 8904299**

(43) Date de publication de la demande : **03.10.90 Bulletin 90/40**

(45) Mention de la délivrance du brevet : **27.10.93 Bulletin 93/43**

(84) Etats contractants désignés : **AT BE CH DE DK FR GB IT LI NL SE**

(56) Documents cités :
**FR-A- 2 249 865**
**GB-A- 2 025 957**
**CHEMICAL ABSTRACTS, vol. 56, résumé no. 8673g, Columbus, Ohio, US; I.K. KOROBITSYNA et al.: "Furanidinobenzopyrylium salts"**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Lagrange, Alain**
**29 rue Auguste Renoir**
**F-78400 Chatou (FR)**
Inventeur : **Lang, Gérard**
**44 avenue Lacour**
**F-95210 Saint-Gratien (FR)**
Inventeur : **Deflandre, André**
**Route de Manon**
**F-60560 Orry-la-Ville (FR)**
Inventeur : **Luppi, Bernadette**
**43, rue Berlioz**
**F-93270 Sevran (FR)**

(74) Mandataire : **Casalonga, Axel et al**
**BUREAU D.A. CASALONGA - JOSSE**
**Morassistrasse 8**
**D-80469 München (DE)**

EP 0 390 683 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés de benzyl-cyclanones, leur procédé de préparation et leur utilisation dans des compositions cosmétiques à usage quotidien ou antisolaire et dans des compositions pharmaceutiques pour le traitement des inflammations et allergies cutanées.

Au cours de ses recherches, la demanderesse vient de découvrir que les dérivés de benzyl-cyclanones ayant la formule suivante :

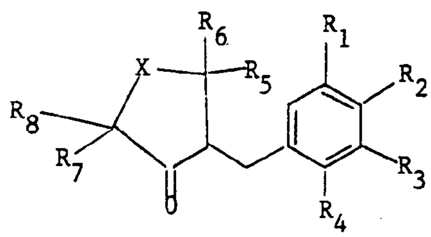

(I)

dans laquelle :

$R_1$ et $R_3$, identiques ou différents, représentent un atome d'hydrogène, un radical hydroxyle, un reste alkyle linéaire ou ramifié en $C_1$-$C_8$ ou un reste alcoxy linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle,

$R_5$, $R_5$, $R_7$ et $R_8$, identiques ou différents, représentent un atome d'hydrogène, un reste alkyle linéaire ou ramifié en $C_1$-$C_{18}$, un reste aralkyle tel que benzyle non substitué ou substitué par un reste alkyle en $C_1$-$C_4$ ou alcoxy en $C_1$-$C_4$, un reste aryle tel que phényle non substitué ou substitué par un reste alkyle en $C_1$-$C_4$; les substituants $R_5$ et $R_5$ et/ou les substituants $R_7$ et $R_8$ peuvent former, avec l'atome de carbone du cycle auquel ils sont rattachés, un cycle saturé contenant de 5 à 12 atomes de carbone non substitué ou substitué par un ou plusieurs restes alkyle linéaires ou ramifiés en $C_1$-$C_8$,

X représente soit un atome d'oxygène, soit un radical $-(CR_9R_{10})_n$ dans lequel n est égal à 1 ou 2 et $R_9$ et $R_{10}$ représentent un atome d'hydrogène ou un reste méthyle; dans le cas où X représente un radical $-(CR_9R_{10})_n$, les substituants $R_5$ et $R_8$ forment avec les atomes du cycle auxquels ils sont rattachés, un système bicyclique contenant 7 ou 8 atomes de carbone; de plus, lorsque X représente un radical $-(CR_9R_{10})_n$- dans lequel n est égal à 1, $R_9$ et $R_{10}$ représentant un reste méthyle, $R_5$ représentant un atome d'hydrogène et $R_5$ et $R_5$ formant avec les atomes du cycle auxquels ils sont rattachés un système bicyclique contenant 7 atomes de carbone, $R_7$ peut également représenter un reste $-CH_2-SO_3H$, éventuellement sous forme de sel de métal alcalin, alcalino-terreux ou d'amine,

présentent, de manière inattendue, d'excellentes propriétés anti-oxydantes vis-à-vis de la peroxydation des lipides polyinsaturés et également vis-à-vis des substances susceptibles de subir des réactions d'oxydation thermo- ou photoinduites (telles que des protéines, des sucres, des pigments, des vitamines, des polymères...).

Or, on sait que la peroxydation des lipides implique la formation de radicaux libres intermédiaires qui endommagent les membranes cellulaires se composant, entre autres, de phospholipides et sont responsables notamment des phénomènes de vieillissement de la peau (A.L TAPPEL dans "Federation Proceedings" Vol. 32, No 8, Août 1973).

Par leurs propriétés anti-oxydantes, les composés de l'invention peuvent donc permettre de mieux lutter contre le vieillissement prématuré de la peau dû à la peroxydation des lipides cutanés.

Ils peuvent aussi permettre d'assurer une meilleure conservation des compositions cosmétiques ou pharmaceutiques comportant une phase grasse en évitant le rancissement des lipides insaturés qui y sont contenus et qui peuvent être d'origine animale comme la lanoline, la cétine (blanc de baleine), la cire d'abeille, le perhydrosqualène, l'huile de tortue, ou d'origine végétale comme l'huile d'olive, l'huile de ricin, l'huile de maïs, l'huile d'amande douce, l'huile d'avocat, l'huile de karité, l'huile de tournesol, l'huile de soja, l'huile d'arachide, les huiles de coprah ou de palmiste, des acides gras essentiels comme la vitamine F et certaines huiles essentielles présentes dans les parfums comme l'huile de citron ou de lavande.

Ils peuvent également permettre d'éviter la dégradation oxydative de composés actifs contenus dans les compositions pharmaceutiques (vitamine A, caroténoïdes...).

La demanderesse a également découvert de manière extrêmement surprenante que les composés de formule (I) ci-dessus pouvaient être utilisés pour le traitement des inflammations et allergies cutanées.

Outre leurs bonnes propriétés anti-oxydantes, les composés selon l'invention présentent une très bonne

stabilité thermique.

Ces composés présentent également l'avantage de ne pas être toxiques ou irritants et d'avoir une parfaite innocuité vis-à-vis de la peau.

La présente invention a pour objet les composés nouveaux de formule (I) suivante :

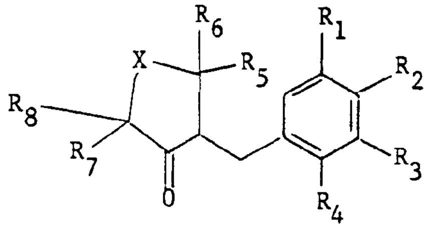

(I')

dans laquelle :

$R_1$ et $R_3$, identiques ou différents, représentent un atome d'hydrogène, un radical hydroxyle, un reste alkyle linéaire ou ramifié en $C_1$-$C_8$ ou un reste alcoxy linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle,

$R_5$, $R_5$, $R_7$ et $R_8$, identiques ou différents, représentent un atome d'hydrogène, un reste alkyle linéaire ou ramifié en $C_1$-$C_{18}$, un reste aralkyle tel que benzyle non substitué ou substitué par un reste alkyle en $C_1$-$C_4$ ou alcoxy en $C_1$-$C_4$, un reste aryle tel que phényle non substitué ou substitué par un reste alkyle en $C_1$-$C_4$; les substituants $R_5$ et $R_5$ et/ou les substituants $R_7$ et $R_8$ peuvent former, avec l'atome de carbone du cycle auquel ils sont rattachés, un cycle saturé contenant de 5 à 12 atomes de carbone non substitué ou substitué par un ou plusieurs restes alkyle linéaires ou ramifiés en $C_1$-$C_8$,

X représente soit un atome d'oxygène, soit un radical $-(CR_9R_{10})_n$ dans lequel n est égal à 1 ou 2 et $R_9$ et $R_{10}$ représentent un atome d'hydrogène ou un reste méthyle; dans le cas où X représente un radical $-(CR_9R_{10})_n$, les substituants $R_6$ et $R_8$ forment avec les atomes du cycle auxquels ils sont rattachés un système bicyclique contenant 7 ou 8 atomes de carbone; de plus, lorsque X représente un radical $-(CR_9R_{10})_n$- dans lequel n est égal à 1, $R_9$ et $R_{10}$ représentant un reste méthyle, $R_5$ représentant un atome d'hydrogène et $R_5$ et $R_5$ formant avec les atomes du cycle auxquels ils sont rattachés un système bicyclique contenant 7 atomes de carbone, $R_7$ peut également représenter un reste $-CH_2-SO_3H$, éventuellement sous forme de sel de métal alcalin, alcalino-terreux ou d'amine, sous réserve que lorsque $R_5$, $R_5$, $R_7$ et $R_5$ désignent un radical méthyle, X un atome d'oxygène, $R_2$ un atome d'hydrogène et $R_4$ un radical hydroxyle, l'un au moins des radicaux $R_1$ et $R_3$ désigne un radical hydroxyle, alkyle ou alcoxy.

Parmi les composés préférés de formule générale (I'), on peut citer :

- la 3',4',5'-trihydroxy-4-benzyl-tétrahydro-2,2,5,5-tétraméthylfurane-3-one,
- la 3',5'-ditert.butyl-4'-hydroxy-4-benzyl-tétrahydro-2,2,5,5-tétraméthyl-furane-3-one,
- la 3',5'-diisopropyl-4'-hydroxy-4-benzyl-tétrahydro-2,2,5,5-tétraméthyl-furane-3-one,
- le 3',5'-ditert.butyl-4'-hydroxy-3-benzyl-camphre,
- le 3',5'-diméthoxy-4'-hydroxy-3-benzyl-camphre,
- le 3',5'-diméthyl-4'-hydroxy-3-benzyl-camphre
- le 3',5'-ditert.-butyl-4'-hydroxy-3-benzyl-nor-camphre,
- le 3',4',5'-trihydroxy-3-benzyl-camphre,
- le 3',5'-di-isopropyl-4'-hydroxy-3-benzyl-camphre,
- le 3'-méthyl-4'-hydroxy-5'-tert.-butyl-3-benzyl-camphre.

Les composés de formule (I') peuvent être obtenus selon plusieurs procédés :

I. <u>Premier procédé</u>

Ce procédé consiste à réduire une benzylidène cyclanone de formule (II) selon le schéma réactionnel ci-dessous :

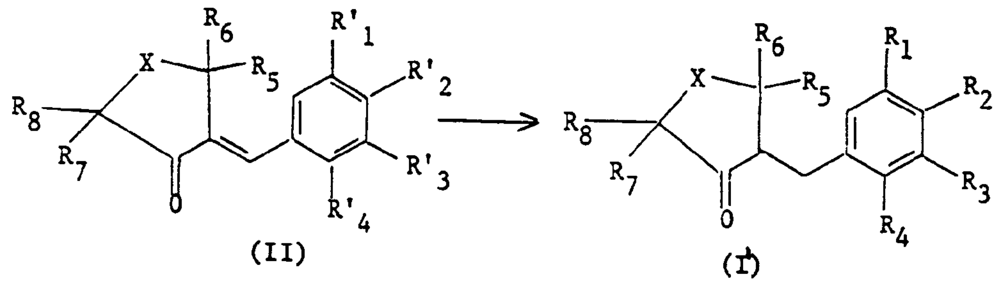

(II) (I')

Dans le composé de formule (II), les substituants $R_5$, $R_5$, $R_7$, $R_5$ et X ont les significations indiquées ci-dessus pour le composé de formule (I'), $R'_1$, $R'_2$, $R'_3$ et $R'_4$ pouvant respectivement représenter $R_1$, $R_2$, $R_3$ ou $R_4$ ou bien un reste arylalcoxy.

La réduction peut être réalisée soit par hydrogénation catalytique, soit par hydrogénation en présence d'un agent de transfert d'hydrogène dans les conditions décrites par G. Brieger et R. J. Nestrick dans Chemical Reviews Vol. 74 (N° 5) pages 567 à 580, par exemple, au moyen de cyclohexène ou d'acide formique en présence d'un catalyseur tel que le palladium sur charbon, éventuellement en présence d'un solvant inerte. La réaction est effectuée à une température comprise entre -78°C et le point d'ébullition du mélange réactionnel.

Lorsque dans la formule (I'), la cyclanone représente un reste camphre, on obtient un mélange d'isomères (endo et exo). On peut orienter la formation de l'isomère le plus stable, en traitant le mélange dans l'alcool, en milieu basique de préférence avec de la potasse ou du méthylate de sodium et en chauffant éventuellement.

Les composés de formule (II) sont obtenus par condensation d'un aldéhyde aromatique de formule (III)

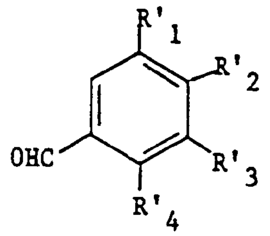

(III)

sur une cyclanone de formule :

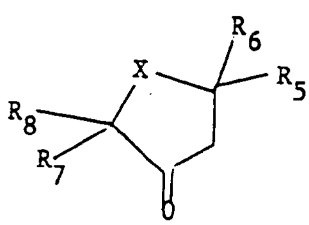

(IV)

Les aldéhydes de formule (III) et les cyclanones de formule (IV) dans lesquels les substituants $R'_1$ à $R'_4$, $R_5$ à $R_8$ et X ont les significations mentionnées ci-dessus pour le composé de formule (II), sont des composés connus.

La condensation de l'aldéhyde (III) sur la cyclanone (IV) peut être effectuée selon l'une des deux méthodes suivantes :

a) Première méthode :

La condensation est effectuée en présence d'un alcoolate de métal alcalin tel que le méthylate de sodium ou le tertiobutylate de potassium, dans un solvant tel que le toluène, le 1,2-diméthoxyéthane ou le 1,2-diéthoxyéthane, à une température comprise entre -78°C et le point d'ébullition du solvant. La condensation peut également être effectuée en présence d'une base minérale telle qu'un hydroxyde, un amidure ou un hydrure de métal alcalin, dans un solvant tel que le 1,2-diméthoxyéthane ou le toluène, à une température comprise entre 0°C et le point d'ébullition du mélange réactionnel.

b) Deuxième méthode :

La condensation de l'aldéhyde (III) sur la cyclanone (IV) est effectuée en présence d'un borane de formule (V) suivante :

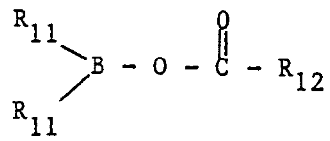

(V)

dans laquelle $R_{11}$ et $R_{12}$ représentent un reste alkyle en $C_1$-$C_6$. Ce composé est obtenu selon le mode opératoire décrit par L.H. TOPORCER and al., J. Am. Chem. Soc. 87, 1236 (1965). Son isolement et sa purification ne sont pas nécessaires pour réaliser la condensation de l'aldéhyde (III) sur la cyclanone (IV).

La réaction de condensation est effectuée à une température de 150°C environ, sans solvant.

II. Deuxième procédé

Ce procédé consiste à condenser une base de Mannich de formule (VI) :

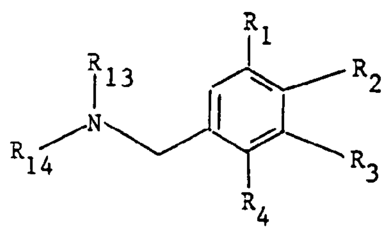

(VI)

sur une cyclanone de formule (IV) ci-dessus. Les bases de Mannich de formule (VI) dans lesquelles les substituants $R_1$ à $R_4$ ont les significations mentionnées ci-dessus pour les composés de formule (I') et $R_{13}$ et $R_{14}$ représentent un reste alkyle en $C_1$-$C_4$ ou bien $R_{13}$ et $R_{14}$ forment, avec l'atome d'azote auxquels ils sont rattachés, un hétérocycle contenant 5 ou 6 atomes, sont des composés connus.

La condensation est effectuée en présence d'un alcoolate de métal alcalin tel que le méthylate de sodium ou le tert.-butylate de potassium, dans un solvant tel que l'éthylène glycol, le 1,2-diméthoxyéthane ou le 1,2-diéthoxyéthane, à une température comprise entre -78°C et le point d'ébullition du solvant.

Les composés de formule (I') peuvent être isolés sous forme d'isomères optiques purs ou bien sous forme de mélange racémique.

La présente invention a donc également pour objet le procédé de préparation des composés nouveaux de formule (I').

Un autre objet de l'invention est une composition cosmétique comprenant, dans un support cosmétiquement acceptable, une quantité efficace d'au moins un dérivé de benzyl-cyclanone de formule (I) ci-dessus.

La composition cosmétique de l'invention peut être utilisée comme composition protectrice de l'épiderme humain ou des cheveux.

La présente invention a également pour objet un procédé de protection de la peau ou des cheveux naturels ou sensibilisés, consistant à appliquer sur ceux-ci une quantité efficace d'au moins un composé de formule (I) contenu dans un support cosmétiquement acceptable.

On entend par "cheveux sensibilisés", des cheveux ayant subi un traitement de permanente, de coloration ou de décoloration.

L'invention a également pour objet une composition cosmétique colorée ou non colorée, stabilisée à l'oxydation, comprenant une quantité efficace d'au moins un dérivé de benzyl-cyclanone de formule (I) ci-dessus.

Lorsqu'elle est utilisée comme composition destinée à protéger l'épiderme humain, la composition cosmétique selon l'invention peut se présenter sous les formes les plus diverses habituellement utilisées pour ce type de composition. Elle peut notamment se présenter sous forme de lotions, d'émulsions telles qu'une crème ou un lait, de gels oléoalcooliques ou hydroalcooliques, de bâtonnets solides, ou être conditionnée en aérosol pour former un spray ou une mousse.

Elle peut contenir les adjuvants cosmétiques habituellement utilisés dans ce type de composition tels que des épaississants, des adoucissants, des humectants, des tensio-actifs, des conservateurs, des anti-mousses,

des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (I), est présent dans des proportions en poids comprises entre 0,1 et 8% et de préférence 0,1 et 5%, par rapport au poids total de la composition cosmétique protectrice de l'épiderme humain.

Une forme de réalisation de l'invention est une émulsion sous forme de crème ou de lait protecteurs comprenant en plus du composé de formule (I), des alcools gras, des esters d'acides gras et notamment des triglycérides d'acides gras, des acides gras, de la lanoline, des huiles ou cires naturelles ou synthétiques et des émulsionnants, en présence d'eau.

La composition cosmétique de l'invention peut également être un gel hydroalcoolique ou oléoalcoolique comprenant un ou plusieurs alcools ou polyols inférieurs tels que l'éthanol, le propylèneglycol ou la glycérine et un épaississant. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

La présente invention vise également les compositions cosmétiques anti-solaires contenant au moins un composé de formule (I) et des filtres UV-B et UV-A.

Dans ce cas, la quantité de composé de formule (I) est comprise entre 0,1 et 8% en poids, la quantité totale de filtres présents dans la composition anti-solaire étant comprise entre 0,5 et 15% en poids par rapport au poids total de la composition anti-solaire.

Lorsque la composition cosmétique selon l'invention est destinée à protéger les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de laque pour cheveux, de composition de permanente, de coloration ou décoloration des cheveux.

Elle contient 0,25 à 8% en poids de composé de formule (I).

La présente invention vise également les compositions cosmétiques contenant au moins un composé de formule (I) à titre d'agent anti-oxydant, constituées par des compositions capillaires telles que les laques pour cheveux, les lotions de mise en plis éventuellement traitantes ou démêlantes, les shampooings, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à l'oxydation au cours du stockage.

De telles compositions contiennent 0,1 à 8% en poids de composé de formule (I).

L'invention vise également un procédé de protection des compositions cosmétiques contre l'oxydation, consistant à incorporer à ces compositions une quantité efficace d'au moins un composé de formule (I).

Un autre objet de l'invention est l'utilisation en tant qu'agents anti-oxydants des composés de formule (I).

L'effet anti-oxydant de ces composés peut être mis en évidence par chimiluminescence d'homogénats cellulaires. Cette technique est basée sur l'émission spontanée de lumière qui résulte de la désactivation radiative des produits de décomposition des lipides peroxydés. L'étude cinétique s'effectue sur des broyats de cervelles de rats, dilués dans un tampon phosphate et est menée parallèlement avec une solution témoin ne contenant pas d'antioxydant et des solutions contenant l'antioxydant à différentes concentrations, variant de $10^{-7}$ à $10^{-5}$ molaire.

Par référence au témoin, on peut déterminer les concentrations $C_x$ en antioxydant permettant l'inhibition de x% de la peroxydation.

L'invention a également pour objet l'application à titre de produits cosmétiques des composés de formule (I).

Comme on l'a indiqué ci-dessus, la demanderesse a en outre découvert au cours de ses recherches, que les composés de formule (I) présentaient une activité pharmacologique intéressante dans le domaine du traitement des inflammations et allergies cutanées.

L'invention a donc pour objet le composé de formule (I) pour son utilisation comme médicament.

L'invention a également pour objet une composition pharmaceutique contenant une quantité efficace d'au moins un composé de formule (I) à titre d'ingrédient actif, dans un support ou un excipient non toxique.

La composition pharmaceutique conforme à l'invention peut être administrée par voie orale ou par voie topique.

Pour la voie orale, la composition pharmaceutique peut se présenter sous forme de comprimés, de gélules, de dragées, de sirops, de suspensions, de solutions, d'émulsions etc.. Pour la voie topique, la composition

pharmaceutique selon l'invention se présente sous forme d'onguent, de crème, de pommade, de solution, de lotion, de gel, spray, suspension, etc.

Cette composition médicamenteuse peut contenir des additifs inertes ou pharmacodynamiquement actifs et notamment : des agents hydratants, des antibiotiques, des agents anti-inflammatoires stéroïdiens ou non stéroïdiens, des caroténoïdes, des agents anti-psoriasiques.

Cette composition peut également contenir des agents d'amélioration de la saveur, des agents conservateurs, des agents stabilisants, des agents régulateurs d'humidité, des agents régulateurs de pH, des agents modificateurs de la pression osmotique, des agents émulsionnants, des anesthésiques locaux, des tampons etc.

Elle peut aussi être conditionnée sous des formes retard ou à libération progressive connues en elles-mêmes.

Le composé de formule (I) conforme à l'invention est présent dans les compositions pharmaceutiques dans des proportions comprises entre 0,01 et 80 % en poids, par rapport au poids total de la composition et de préférence entre 0,1 et 20 % en poids.

Dans l'application thérapeutique, le traitement est déterminé par le médecin et peut varier selon l'âge, le poids et la réponse du patient ainsi que la gravité des symptômes.

Lorsque les composés de formule (I) sont administrés par voie orale, le dosage est généralement compris entre 0,1 et 50 mg/kg/jour et de préférence entre 0,2 et 20 mg/kg/jour. La durée du traitement est variable suivant la gravité des symptômes et peut s'étaler entre 1 et 25 semaines, de façon continue ou discontinue.

Les compositions par voie topique contiennent de préférence de 0,25 % à 8 % en poids de composé de formule (I).

Comme support ou excipient de la composition pharmaceutique de l'invention, on peut utiliser tous supports ou excipients conventionnels non toxiques.

Les exemples suivants sont destinés à illustrer l'invention sans pour autant présenter un caractère limitatif.

## EXEMPLES DE PREPARATION

### EXEMPLE 1

Préparation du composé de formule (I) dans laquelle $R_1$=$R_2$=$R_3$=OH, $R_4$=H, $R_5$=$R_6$=$R_7$=$R_8$=$CH_3$ et X=O :

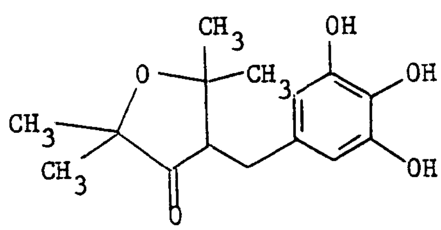

1) Préparation de la 3',4',5'-tribenzyloxy-4-benzylidène-tétrahydro-2,2,5,5-tétraméthylfurane-3-one :

A une solution de 14,2 g (0,1 mole) de tétrahydro-2,2,5,5-tétraméthyl-furane-3-one dans 20 $cm^3$ de 1,2-diméthoxyéthane on ajoute, entre 5 et 10°C, 5,4 g (0,1 mole) de méthylate de sodium, puis 39,8 g (0,094 mole) de 3,4,5-tribenzyloxy-4-benzaldéhyde dans 100 $cm^3$ de 1,2-diméthoxyéthane. Le mélange réactionnel est agité pendant 30 minutes à une température voisine de 0°C puis on verse le mélange réactionnel dans une solution diluée d'acide chlorhydrique. Le précipité est filtré, lavé abondamment à l'eau puis à l'heptane chaud et séché sous pression réduite. On obtient 35,8 g de produit attendu possédant les caractéristiques suivantes :

- Point de fusion : 73-75°C
- Analyse élémentaire : $C_{36}H_{36}O_5$

| | C % | H % | O % |
|---|---|---|---|
| Calculé | 78,81 | 6,61 | 14,58 |
| Trouvé | 78,78 | 6,61 | 14,64 |

- Spectre UV ($CH_2Cl_2$)

. $\lambda_{max}$ : 340 nm
. $\varepsilon_{max}$ : 11400

2) Préparation de la 3',4',5'-trihydroxy-4-benzyl-tétrahydro-2,2,5,5-tétraméthylfurane-3-one

On chauffe au reflux pendant une heure 5 g (0,009 mole) de 3',4',5'-tribenzyloxy-4-benzylidène-tétrahydro-2,2,5,5-tétraméthylfurane-3-one obtenue ci-dessus dans 50 cm³ d'un mélange 50/50 d'éthanol et de tétrahydrofuranne contenant 5 g de formiate d'ammonium et 0,5 g d'hydroxyde de palladium. On laisse refroidir puis on filtre le mélange réactionnel sur Célite. Le gâteau de Célite est lavé par un mélange 50/50 d'éthanol et de tétrahydrofuranne. Le solvant est distillé sous pression réduite et le résidu est recristallisé dans l'éthanol à 50%.

Le produit obtenu possède les caractéristiques suivantes :

- Point de fusion : 186°C
- Analyse élémentaire : $C_{15}H_{20}O_5$

| | C% | H% | O% |
|---|---|---|---|
| Calculé | 64,27 | 7,19 | 28,54 |
| Trouvé | 64,54 | 6,60 | 28,76 |

EXEMPLE 2

Préparation du composé de formule (I) dans laquelle $R_1=R_3$=tert.-butyle, $R_2$=OH, $R_4$=H, $R_5=R_6=R_7=R_8=CH_3$ et X=O :

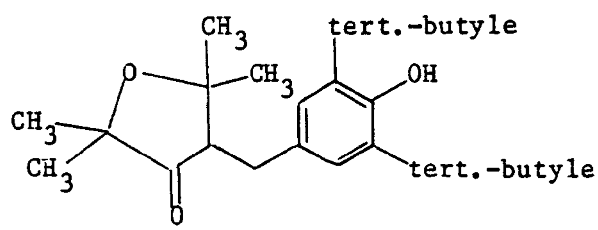

1) Préparation de la 3',5'-ditert.-butyl-4'-hydroxy-4-benzylidène-tétrahydro-2,2,5,5-tétraméthylfurane-3-one :

A 100 cm³ de solution 1M de triéthylborane dans l'hexane, on ajoute à 0°C 8,2 g (0,08 mole) d'acide pivalique. Après 15 minutes d'agitation, on laisse revenir à température ambiante, puis on ajoute 5,4 g (0,038 mole) de tétrahydro-2,2,5,5-tétraméthyl-furane-3-one et 8,9 g (0,038 mole) de 3,5-ditert.-butyl-4-hydroxy-benzaldéhyde. L'hexane est distillé et on chauffe à 150°C pendant 3 heures. On distille les produits volatils sous pression réduite (2 KPa puis 13 Pa). Après recristallisation du résidu dans un mélange éthanol-eau, on obtient 5,5 g de produit attendu sous forme de cristaux jaune pâle possédant les caractéristiques suivantes :

- Point de fusion : 146°C
- Analyse élémentaire : $C_{23}H_{34}O_3$

| | C% | H% | O% |
|---|---|---|---|
| Calculé | 77,05 | 9,56 | 13,39 |
| Trouvé | 76,65 | 9,56 | 13,78 |

- Spectre UV ($CH_2Cl_2$)
  . $\lambda_{max}$ : 335 nm
  . $\varepsilon_{max}$ : 18170

2) Préparation de la 3'5'-ditert.-butyl-4'-hydroxy-4-benzyl-tétrahydro-2,2,5,5-tétraméthylfurane-3-one :

On met en suspension, sous argon, 3 g (0,0083 mole) de 3',5'-ditert.-butyl-4'-hydroxy-4-benzylidène-tétrahydro-2,2,5,5-tétraméthyl-furane-3-one obtenue ci-dessus dans 50 $cm^3$ d'acide formique. On ajoute 4 g de palladium sur charbon contenant 50% d'eau (5% en poids de palladium par rapport au charbon). On agite pendant 1 heure à température ambiante puis on filtre sur Célite après dilution par 50 $cm^3$ d'eau. Le gâteau de Célite est lavé plusieurs fois au dichlorométhane. La phase organique est décantée puis séchée sur sulfate de sodium. Le solvant est distillé sous pression réduite. Après recristallisation du résidu dans l'éther diisopropylique, on obtient 0,8 g de produit attendu sous forme de cristaux blancs possédant les caractéristiques suivantes :

- Point de fusion : 104°C
- Analyse élémentaire : $C_{23}H_{36}O_3$

| | C% | H% | 0% |
|---|---|---|---|
| Calculé | 76,62 | 10,06 | 13,31 |
| Trouvé | 76,69 | 10,18 | 13,50 |

EXEMPLE 3

Préparation du composé de formule (I) dans laquelle $R_1$=$R_3$=tert.-butyle, $R_2$=OH, $R_4$=H, $R_5$=H, $R_6$-$R_8$= $-CH_2-CH_2-$, $R_7$=$CH_3$ et X= $-C(CH_3)_2-$ :

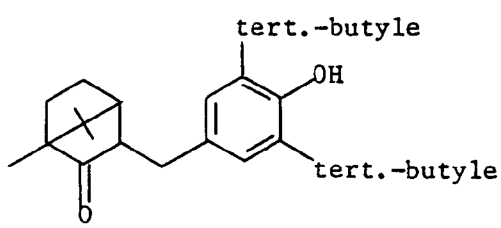

1) Préparation du 3',5'-ditert.-butyl-4'-hydroxy-3-benzylidène-camphre :

Ce composé est préparé selon le mode opératoire décrit dans la première partie de l'exemple 2. La tétrahydro-2,2,5,5-tétraméthylfurane-3-one est remplacée par le d,1-camphre.

Le produit attendu est obtenu sous forme de cristaux blancs après recristallisation dans l'éther diisopropylique. Il possède les caractéristiques suivantes :

- Point de fusion : 158°C
- Analyse élémentaire : $C_{25}H_{36}O_2$

| | C% | H% | 0% |
|---|---|---|---|
| Calculé | 81,47 | 9,84 | 8,60 |
| Trouvé | 81,37 | 9,84 | 8,78 |

- Spectre UV ($CHCl_3$)
  - . $\lambda_{max}$ : 323 nm
  - . $\varepsilon_{max}$ : 24200

2) Préparation du 3',5'-ditert.-butyl-4'-hydroxy-3-benzyl-camphre

2a) Préparation d'un mélange d'isomères majoritairement exo

On met en suspension, sous argon, 3 g (0,008 mole) de 3',5'-ditert.-butyl-4'-hydroxy-3-benzylidène-camphre obtenu ci-dessus dans 50 $cm^3$ d'acide formique. On ajoute 4 g de palladium sur charbon contenant 50% d'eau (5% en poids de palladium par rapport au charbon). On agite pendant 1 heure à température ambiante puis on filtre sur Célite après dilution par 50 $cm^3$ d'eau. Le gâteau de Célite est lavé plusieurs fois au dichlorométhane. La phase organique est décantée puis séchée sur sulfate de sodium. Le solvant est distillé sous

pression réduite. Après recristallisation du résidu dans l'éther diisopropylique, on obtient 1,9 g de produit attendu sous forme de cristaux blancs, constitué d'un mélange d'isomères comprenant en majorité la forme exo, possédant les caractéristiques suivantes :

- Point de fusion : 166°C
- Analyse élémentaire : $C_{25}H_{38}O_2$

| | C% | H% | O% |
|---|---|---|---|
| Calculé | 81,03 | 10,34 | 8,64 |
| Trouvé | 80,81 | 10,14 | 9,17 |

2b) Préparation d'un mélange d'isomères majoritairement endo

Le mélange d'isomères ci-dessus (1,9 g) est mis en solution dans 14 cm³ d'éthanol en présence de 0,5 g de méthylate de sodium. Le mélange est chauffé, sous argon, au reflux pendant 2 heures. Après quoi, le milieu réactionnel est acidifié par de l'acide chlorhydrique 2N; le produit attendu précipite; on le filtre. Après recristallisation du précipité dans un milieu éthanol/eau (90/10) on obtient des cristaux blancs constitués d'un mélange d'isomères contenant majoritairement la forme endo, possédant les caractéristiques suivantes :

- Point de fusion : 166°C
- Analyse élémentaire : $C_{25}H_{38}O_2$

| | C% | H% | O% |
|---|---|---|---|
| Calculé | 81,03 | 10,34 | 8,64 |
| Trouvé | 81,13 | 10,38 | 8,68 |

Les deux formes endo et exo ont les mêmes caractéristiques physicochimiques.

EXEMPLE 4

Préparation d'un composé de formule (I) dans laquelle $R_1$=$CH_3$, $R_2$=OH, $R_3$=tert.-butyle, $R_4$=$R_5$=H, $R_6$-$R_8$= $-CH_2CH_2-$,$R_7$=$CH_3$ et X= $-C(CH_3)_2-$ :

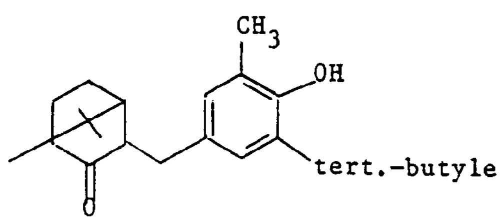

1) Préparation du 3'-tert.-butyl-4'-hydroxy-5'-methyl-3-benzylidène-camphre

On chauffe au reflux pendant 30 minutes, 10,5 g (0,068 mole) de d,l-camphre et 15,2 g (0,136 mole) de tert.-butylate de potassium dans 100 ml de toluène. On ajoute à 80°C 13 g (0,068 mole) de 3-tert.-butyl-4-hydroxy-5-méthyl-benzaldéhyde puis on distille 80 cm³ de solvant. Après 1 heure de chauffage, le mélange réactionnel est refroidi à température ambiante puis versé dans une solution aqueuse d'acide chlorhydrique. On extrait au dichlorométhane. La phase organique est lavée à l'eau, séchée sur sulfate de sodium, puis évaporée à sec.

Le produit est dispersé dans l'heptane.

On obtient 4,1 g de produit attendu sous forme de cristaux très légèrement beige possédant les caractéristiques suivantes :

- Point de fusion : 152°C
- Analyse élémentaire : $C_{22}H_{30}O_2$

| | C% | H% |
|---|---|---|
| Calculé | 80,94 | 9,26 |
| Trouvé | 80,97 | 9,26 |

- Spectre UV ($CH_2Cl_2$)
  - . $\lambda_{max}$ = 319 nm
  - . $\varepsilon_{max}$ = 23280

2) Préparation du 3'-tert.-butyl-4'-hydroxy-5'-méthyl-3-benzyl-camphre :

Ce composé est préparé selon le mode opératoire décrit dans la deuxième partie de l'exemple 3.

Le produit attendu est obtenu sous forme de cristaux blancs après recristallisation dans l'heptane. Il possède les caractéristiques suivantes :

- Point de fusion : 147°C
- Analyse élémentaire : $C_{25}H_{36}O_2$

| | C% | H% | O% |
|---|---|---|---|
| Calculé | 80,44 | 9,82 | 9,74 |
| Trouvé | 80,00 | 9,88 | 9,98 |

EXEMPLE 5

Préparation du composé de formule (I) dans laquelle : $R_1$=$R_3$=tert.-butyle, $R_2$=OH,$R_4$=H,$R_5$=H,$R_6$-$R_8$=-$CH_2$-$CH_2$-,$R_7$= H et X =-$CH_2$-

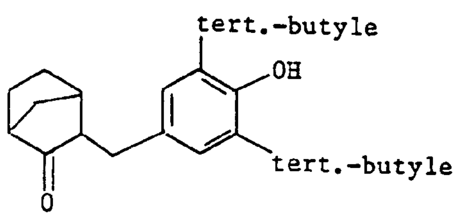

1) Préparation du 3',5'-ditert.-butyl-4'-hydroxy-3-benzylidène-nor camphre :

Ce composé est préparé selon le mode opératoire décrit dans la première partie de l'exemple 2. La tétrahydro-2,2,5,5-tétraméthylfurane-3-one, est remplacée par le nor-camphre.

Le produit attendu est obtenu sous forme de cristaux blancs après recristallisation dans l'heptane; il possède les caractéristiques suivantes :

- Point de fusion : 134°C
- Analyse élémentaire : $C_{25}H_{36}O_2$

| | C% | H% | O% |
|---|---|---|---|
| Calculé | 80,94 | 9,26 | 9,80 |
| Trouvé | 81,07 | 9,34 | 10,06 |

- Spectre UV ($CH_2Cl_2$)
  - . $\lambda_{max}$ : 323 nm
  - . $\varepsilon_{max}$ : 24350

2) Préparation du 3',5'-ditert.-butyl-4'-hydroxy-3-benzyl nor-camphre

Ce composé est préparé selon le mode opératoire décrit dans la deuxième partie 2a) de l'exemple 3.

Le produit attendu est obtenu sous forme de cristaux blancs, après recristallisation dans l'éther diisopropylique. Il possède les caractéristiques suivantes :

- Point de fusion : 112-114°C
- Analyse élémentaire : $C_{22}H_{32}O_2$

| | C% | H% | 0% |
|---|---|---|---|
| Calculé | 80,44 | 9,82 | 9,74 |
| Trouvé | 79,86 | 9,86 | 10,22 |

EXEMPLE 6

Préparation d'un composé de formule (I) dans laquelle $R_1=R_3=OCH_3, R_2=OH, R_4=R_5=H, R_6-R_8=-CH_2-CH_2-$, $R_7=CH_3$ et $X = -C(CH_3)_2-$

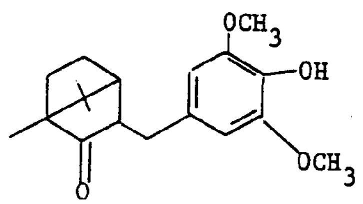

1) Préparation du 3',5'-diméthoxy-4'-hydroxy-3-benzylidène-camphre :

Ce composé est obtenu selon le mode opératoire décrit dans la première partie de l'exemple 4 ci-dessus dans lequel le 3-tert.-butyl-4-hydroxy-5-méthyl-benzaldéhyde est remplacé par le 3,5-diméthoxy-4-hydroxy-benzaldéhyde.

Le produit attendu est obtenu sous forme de cristaux blancs, après recristallisation dans l'éther diisopropylique. Il possède les caractéristiques suivantes :

- Point de fusion : 158°C
- Analyse élémentaire : $C_{19}H_{24}O_4$

| | C% | H% | 0% |
|---|---|---|---|
| Calculé | 72,13 | 7,65 | 20,23 |
| Trouvé | 72,17 | 7,64 | 20,30 |

- Spectre UV (dichlorométhane)
  . $\lambda_{max}$ : 327 nm
  . $\varepsilon_{max}$ : 21300

2) Préparation du 3',5'-diméthoxy-4'-hydroxy-3-benzyl-camphre

Ce composé est préparé selon le mode opératoire décrit dans la deuxième partie 2a) de l'exemple 3.

Le produit attendu est obtenu sous forme de cristaux blancs après recristallisation dans l'éther diisopropylique. Il possède les caractéristiques suivantes :

- Point de fusion : 87°C
- Analyse élémentaire : $C_{19}H_{26}O_4$

| | C% | H% | O% |
|---|---|---|---|
| Calculé | 71,67 | 8,23 | 20,10 |
| Trouvé | 71,57 | 8,23 | 20,18 |

EXEMPLE 7

Préparation d'un composé de formule (I) dans laquelle $R_1$=$R_3$= isopropyle, $R_2$=OH, $R_4$=$R_5$=H, $R_6$-$R_8$=$-CH_2-CH_2-$,$R_7$=$CH_3$ et X=$-C(CH_3)_2-$

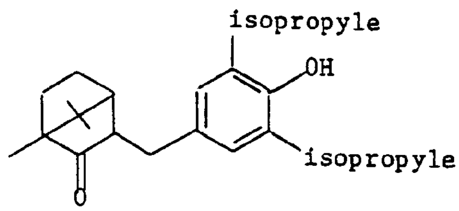

1) Préparation du 3',5'-diisopropyl-4'-hydroxy-3-benzylidène-camphre

Ce composé est obtenu selon le mode opératoire décrit dans la première partie de l'exemple 4 ci-dessus dans lequel le 3-tert.-butyl-4-hydroxy-5-méthyl-benzaldéhyde est remplacé par le 3,5-diisopropyl-4-hydroxy-benzaldéhyde.

Le produit attendu est obtenu sous forme de cristaux blancs après recristallisation dans l'éther diisopropylique. Il possède les caractéristiques suivantes :

- Point de fusion : 159-160°C
- Analyse élémentaire : $C_{23}H_{32}O_2$

| | C% | H% | O% |
|---|---|---|---|
| Calculé | 81,13 | 9,47 | 9,40 |
| Trouvé | 81,08 | 9,41 | 9,66 |

- Spectre UV ($CH_2Cl_2$)
  . $\lambda_{max}$ : 318 nm
  . $\varepsilon_{max}$ : 25300

2) Préparation du 3',5'-diisopropyl-4'-hydroxy-3-benzyl-camphre

Ce composé est préparé selon le mode opératoire décrit dans la deuxième partie 2a) de l'exemple 3.

Le produit attendu est obtenu sous forme de cristaux blancs après recristallisation dans l'éther diisopropylique. Il possède les caractéristiques suivantes :

- Point de fusion : 122°C
- Analyse élémentaire : $C_{23}H_{34}O_2$

| | C% | H% | O% |
|---|---|---|---|
| Calculé | 80,65 | 10,01 | 9,34 |
| Trouvé | 80,64 | 10,02 | 9,46 |

EXEMPLE 8

Préparation d'un composé de formule (I) dans laquelle $R_1$=$R_3$=$CH_3$, $R_2$=OH, $R_4$=$R_5$=H,$R_6$-$R_8$=-$CH_2CH_2$-, $R_7$= $CH_3$ et X= -$C(CH_3)_2$- :

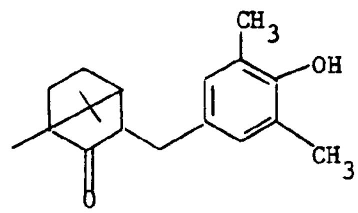

1) Préparation du 3',5'-diméthyl-4'-hydroxy-3-benzylidène-camphre

Ce composé est obtenu selon le mode opératoire décrit dans la première partie de l'exemple 4 ci-dessus dans lequel le 3-tert.-butyl-4-hydroxy-5-méthyl-benzaldéhyde est remplacé par le 3,5-diméthyl-4-hydroxy-benzaldéhyde.

Le produit attendu est obtenu sous forme de cristaux blancs après recristallisation dans l'éther diisopropylique. Il possède les caractéristiques suivantes :

- Point de fusion : 170°C
- Analyse élémentaire : $C_{19}H_{24}O_2$

| | C% | H% | 0% |
|---|---|---|---|
| Calculé | 80,24 | 8,51 | 11,25 |
| Trouvé | 80,21 | 8,50 | 11,28 |

- Spectre UV ($CH_2Cl_2$)
  . $\lambda_{max}$ : 318 nm
  . $\varepsilon_{max}$ : 24100

2) Préparation du 3',5'-diméthyl-4'-hydroxy-3-benzyl-camphre

Ce composé est préparé selon le mode opératoire décrit dans la deuxième partie 2a) de l'exemple 3.

Le produit attendu est obtenu sous forme de cristaux blancs, après recristallisation dans l'heptane. Il possède les caractéristiques suivantes :

- Point de fusion : 150°C
- Analyse élémentaire : $C_{19}H_{26}O_2$

| | C% | H% | 0% |
|---|---|---|---|
| Calculé | 79,68 | 9,15 | 11,17 |
| Trouvé | 79,61 | 9,09 | 11,34 |

EXEMPLE 9

Préparation d'un composé de formule (I) dans laquelle $R_1=R_2=R_3=OH, R_4=R_5=H, R_6-R_8=-CH_2-CH_2-, R_7=CH_3$ et $X =-C(CH_3)_2-$ :

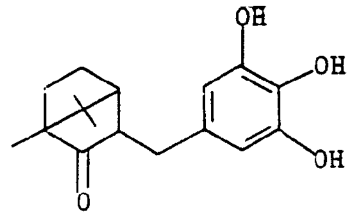

a) Préparation du 3',4',5'-tribenzyloxy-3-benzylidène camphre

Ce composé est préparé selon le mode opératoire décrit dans la première partie de l'exemple 1. La tétrahydro-2,2,5,5-tétraméthylfurane-3-one est remplacée par le d,1-camphre.

Le produit attendu est obtenu sous forme de cristaux blancs, après recristallisation dans l'éthanol à 95°. Il possède les caractéristiques suivantes :

- Point de fusion : 88°C
- Analyse élémentaire : $C_{38}H_{38}O_4$

| | C% | H% | 0% |
|---|---|---|---|
| Calculé | 81,69 | 6,86 | 11,45 |
| Trouvé | 81,60 | 6,87 | 11,51 |

b) Préparation du 3',4',5'-trihydroxy-3-benzyl-camphre

Ce composé est préparé selon le mode opératoire décrit dans la deuxième partie 2a) de l'exemple 3.

Le produit attendu est obtenu sous forme de cristaux blancs après recristallisation dans l'éther diisopropylique. Il possède les caractéristiques suivantes :

- Point de fusion : 153°C
- Analyse élémentaire : $C_{17}H_{22}O_4$

| | C% | H% | 0% |
|---|---|---|---|
| Calculé | 70,32 | 7,64 | 22,04 |
| Trouvé | 70,26 | 7,66 | 22,09 |

EXEMPLE 10

Préparation du composé de formule (I) dans laquelle $R_1=OCH_3$, $R_2=H$, $R_3$=tert.-butyle, $R_4=OH$, $R_5=H$, $R_6-R_8=-CH_2-CH_2-$, $R_7=CH_2-SO_3H$ et $X=-C(CH_3)_2$ :

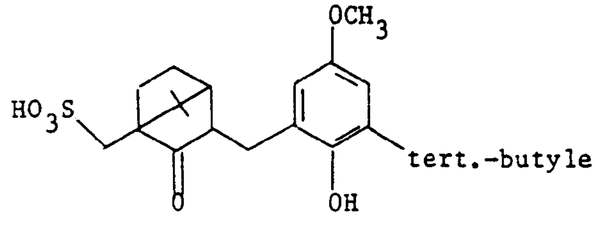

1) Préparation de l'acide 3'-tert.-butyl-2'-hydroxy-5'-méthoxy-3-benzylidène-campho-10-sulfonique

On chauffe au reflux pendant 1 heure 8,8 g (0,038 mole) d'acide d,1-campho-10-sulfonique, 6,1 g (0,114 mole) de méthylate de sodium et 7,9 g (0,038 mole) de 3-tert.-butyl-2-hydroxy-5-méthoxy- benzaldéhyde dans 130 cm[3] de 1,2-diméthoxyéthane. Après refroidissement, on ajoute, sous agitation, 2 équivalents d'acide chlorhydrique 2N. La phase aqueuse est saturée par du chlorure de sodium. On extrait au dichlorométhane, puis le solvant est évaporé. Le solide jaune ainsi obtenu est dispersé à chaud dans l'éther diisopropylique. On filtre puis sèche sous vide. Le sel de sodium ainsi obtenu est redissous dans l'eau et la solution est filtrée sur colonne de résine échangeuse d'ions Dowex 50. On obtient, après évaporation de l'eau sous pression réduite, à température ambiante, le composé attendu sous forme d'un solide orangé possédant les caractéristiques suivantes :

- Analyse élémentaire : $C_{22}H_{30}O_6S$

| | C% | H% | O% | S% |
|---|---|---|---|---|
| Calculé | 62,54 | 7,16 | 22,72 | 7,59 |
| Trouvé | 62,57 | 7,49 | 22,97 | 6,93 |

- Spectre (sous forme de sel de sodium, dans l'eau)
  - . $\lambda_{max1}$ : 295 nm
  - . $\varepsilon_{max1}$ 11150
  - . $\lambda_{max2}$ : 355 nm
  - . $\varepsilon_{max2}$ : 7500

2) Préparation de l'acide 3'-tert.-butyl-2'-hydroxy-5'-méthoxy-3-benzyl-campho-10-sulfonique

Ce composé est préparé selon le mode opératoire décrit dans la deuxième partie 2a) de l'exemple 3.

Le produit attendu est obtenu sous forme de cristaux blancs après recristallisation dans l'éther diisopropylique. Il possède les caractéristiques suivantes :

- Point de fusion ≦ 250°C
- Analyse élémentaire : $C_{22}H_{32}O_6S,1/2H_2O$

| | C% | H% | O% | S% |
|---|---|---|---|---|
| Calculé | 60,96 | 7,62 | 24,01 | 7,39 |
| Trouvé | 61,23 | 7,97 | 24,18 | 6,60 |

EXEMPLE 11

Préparation du composé de formule (I) dans laquelle $R_1$=$R_3$=isopropyle, $R_2$= OH, $R_4$=H, $R_5$=$R_6$=$R_7$=$R_8$=$CH_3$ et X=O

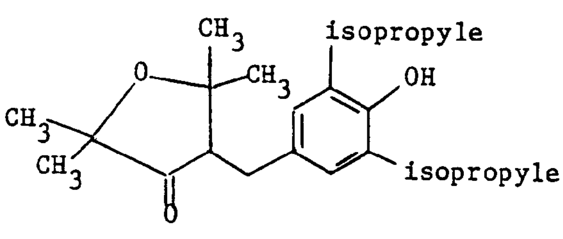

1) Préparation de la 3',5'-diisopropyl-4'-hydroxy-4-benzylidène tétrahydro-2,2,5,5-tétraméthylfurane-3-one :

Ce composé est obtenu selon le mode opératoire décrit dans la première partie de l'exemple 1 ci-dessus dans lequel le 3,4,5-tribenzyloxy-4-benzaldéhyde est remplacé par le 3,5-diisopropyl-4-hydroxy-benzaldéhyde.

Le produit attendu est obtenu sous forme d'un solide blanc. Il possède les caractéristiques suivantes :
- Point de fusion : 142°C
- Analyse élémentaire : $C_{21}H_{30}O_3$

| | C% | H% | O% |
|---|---|---|---|
| Calculé | 76,33 | 9,15 | 14,52 |
| Trouvé | 76,57 | 9,23 | 14,68 |

- Spectre UV ($CH_2Cl_2$)
  . $\lambda_{max}$ : 335 nm
  . $\varepsilon_{max}$ : 22400

2) Préparation de la 3',5'-diisopropyl-4'-hydroxy-4-benzyl-tétrahydro-2,2,5,-tétraméthylfurane-3-one :

Ce composé est préparé selon le mode opératoire décrit dans la deuxième partie de l'exemple 3.

Le produit attendu est obtenu sous forme de cristaux blancs après recristallisation dans l'heptane. Il possède les caractéristiques suivantes :
- Point de fusion : 90°C
- Analyse élémentaire : $C_{21}H_{32}O_3$

| | C% | H% | O% |
|---|---|---|---|
| Calculé | 75,86 | 9,70 | 14,44 |
| Trouvé | 75,81 | 9,77 | 14,44 |

EXEMPLE 12

Préparation du composé de formule (I) dans laquelle $R_1$=$R_3$=tert.-butyle, $R_2$=OH, $R_4$=H, $R_5$=H, $R_6$-$R_8$= $-CH_2-CH_2-$, $R_7$=$CH_3$ et X= $-C(CH_3)_2$

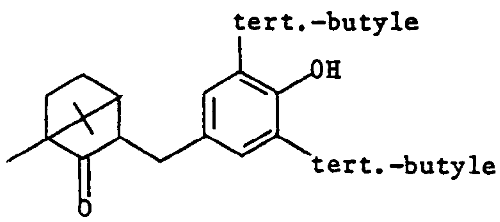

1) Préparation du 3',5'-ditert.-butyl-4'-hydroxy-3-benzylidène camphre :

Ce composé est préparé selon le mode opératoire décrit dans la première partie de l'exemple 2. La tétrahydro-2,2,5,5-tétraméthylfurane-3-one est remplacée par le d-camphre.

Le produit attendu est obtenu sous forme de cristaux blancs après recristallisation dans l'éther diisopropylique. Il possède les caractéristiques suivantes :
- Point de fusion : 186°C
- Analyse élémentaire : $C_{25}H_{36}O_2$

| | C% | H% | O% |
|---|---|---|---|
| Calculé | 81,47 | 9,84 | 8,60 |
| Trouvé | 81,40 | 9,87 | 8,79 |

- Spectre UV ($CHCl_3$)

. $\lambda_{max}$ : 325 nm
. $\varepsilon_{max}$ : 23000

2) Préparation du 3',5'-ditert.-butyl-4'-hydroxy-3-benzyl-camphre (D)

Ce composé est préparé selon le mode opératoire décrit dans la deuxième partie de l'exemple 3.

Le produit attendu est obtenu sous forme de cristaux blancs après recristallisation dans un mélange éthanol/eau 95/5.

Il possède les caractéristiques suivantes :

- Point de fusion : 166°C
- Analyse élémentaire : $C_{25}H_{38}O_2$

| | C% | H% | O% |
|---|---|---|---|
| Calculé | 81,03 | 10,34 | 8,63 |
| Trouvé | 81,22 | 10,29 | 8,79 |

Mise évidence des propriétés antioxydantes de ces composés par chimiluminescence d'homogénats cellulaires

Le test de chimiluminescence est exécuté selon la publication de E.A. LISSI, T. CACERES et L.A. VIDELA "Visible Chemiluminescence from rat brain homogenates undergoing autoxidation. I. Effect of additives and products accumulation" dans "Journal of Free Radicals in Biology and Medicine", Vol.2, pp. 63-69 (1986). Ce test a été simplifié dans le sens où on a mesuré uniquement la chimiluminescence sans mesurer l'accumulation de l'acide thiobarbiturique après 1 heure d'irradiation. Cependant, selon la publication, il y a une bonne corrélation entre la chimiluminescence et la mesure de l'accumulation de l'acide thiobarbiturique.

Des cervelles de rats sont prélevées immédiatement après dislocation des cervicales. Elles sont lavées avec une solution tampon contenant 140 mM de NaCl et 40 mM de phosphate de potassium, débarrassées des vaisseaux apparents et de la membrane extérieure, puis broyées avec 4 fois leur volume de solution tampon. Ce broyat est dilué 3 fois avec la solution tampon (dilution totale 12 fois par rapport aux organes de départ).

10 ml d'homogénat dilué est introduit dans des flacons de comptage contenant 200 µl de solution du produit antioxydant à tester, dissous à différentes concentrations dans du méthanol ou du diméthylsulfoxyde.

Immédiatement après agitation, les flacons sont introduits dans un compteur de scintillation (BECKMAN) et comptés périodiquement.

Parallèlement, une étude est menée sur une solution témoin ne contenant pas d'antioxydant.

On établit ainsi, pour chaque concentration une courbe d'intensité de chimiluminescence en fonction du temps.

Par comparaison des pentes des courbes obtenues avec les solutions à différentes concentrations et la solution témoin, on détermine $C_{50}$, c'est-à-dire la concentration en antioxydant permettant l'inhibition de 50% de la peroxydation.

Composé de l'exemple 2 = $C_{50}$ = $1,10.10^{-6}$M
Composé de l'exemple 3 = $C_{50}$ = $2,00.10^{-6}$M
Composé de l'exemple 4 = $C_{50}$ = $1,40.10^{-6}$M
Composé de l'exemple 5 = $C_{50}$ = $1,50.10^{-6}$M
Composé de l'exemple 6 = $C_{50}$ = $1,25.10^{-6}$M
Composé de l'exemple 7 = $C_{50}$ = $0,80.10^{-6}$M
Composé de l'exemple 9 = $C_{50}$ = $0,80.10^{-6}$M

Par comparaison, la $C_{50}$ du BHT (ditert.-butyl-hydroxy toluène), qui est un antioxydant classique, est $3,20.10^{-6}$M.

Compositions pharmaceutiques utilisées par voie topique

Exemple A - Onguent apaisant

(A appliquer sur une peau irritée pour calmer)

| | | | |
|---|---|---|---|
| - Composé de l'exemple 3 | | 2,0 | g |
| - Huile de vaseline fluide | | 9,1 | g |
| - Silice vendue par la Société DEGUSSA sous le nom de "AEROSIL 200" | | 9,2 | g |
| - Myristate d'isopropyle | qsp | 100 | g |

Exemple B - Crème (huile-dans-l'eau) anti-inflammatoire

| | | | |
|---|---|---|---|
| - Composé de l'exemple 6 | | 2,5 | g |
| - Dodécyl sulfate de sodium | | 0,8 | g |
| - Glycérol | | 2,0 | g |
| - Alcool stéarylique | | 20,0 | g |
| - Triglycérides d'acides caprique/caprylique vendus par la Société DYNAMIT NOBEL sous le nom de "MIGLYOL 812" | | 20,0 | g |
| - Conservateur | qs | | |
| - Eau déminéralisée | qsp | 100 | g |

Exemple C - Gel apaisant

| | | | |
|---|---|---|---|
| - Composé de l'exemple 7 | | 1,5 | g |
| - Hydroxypropyl cellulose vendue par la Société HERCULES sous le nom de "KLUCEL HF" | | 2,0 | g |
| - Ethanol | | 70,0 | g |
| - Eau | qsp | 100 | g |

Composition cosmétique

Exemple D - Gel protecteur de la peau

| | | | |
|---|---|---|---|
| - Composé de l'exemple 1 | | 0,2 | g |
| - Glycérine | | 12,0 | g |
| - Acide polyacrylique réticulé par un agent polyfonctionnel vendu sous la marque "CARBOPOL 934" par la Société GOODRICH | | 0,8 | g |
| - Ethanol | | 15,0 | g |
| - Conservateur, parfum | qs | | |
| - Triéthanolamine | qs | pH : 5,3 | |
| - Eau déminéralisée | qsp | 100 | g |

## Revendications

1. Dérivé de benzyl-cyclanone caractérisé par le fait qu'il répond à la formule :

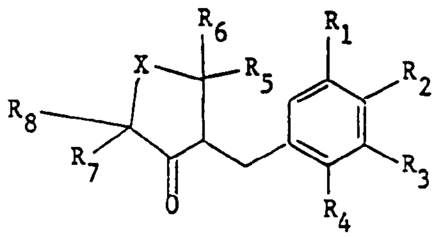

(I')

dans laquelle :

$R_1$ et $R_3$, identiques ou différents, représentent un atome d'hydrogène, un radical hydroxyle, un reste alkyle linéaire ou ramifié en $C_1$-$C_8$ ou un reste alcoxy linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle,

$R_5$, $R_6$, $R_7$ et $R_8$, identiques ou différents, représentent un atome d'hydrogène, un reste alkyle linéaire ou ramifié en $C_1$-$C_{18}$, un reste aralkyle tel que benzyle non substitué ou substitué par un reste alkyle en $C_1$-$C_4$ ou alcoxy en $C_1$-$C_4$, un reste aryle tel que phényle non substitué ou substitué par un reste alkyle en $C_1$-$C_4$; les substituants $R_5$ et $R_6$ et/ou les substituants $R_7$ et $R_6$ peuvent former, avec l'atome de carbone du cycle auquel ils sont rattachés, un cycle saturé contenant de 5 à 12 atomes de carbone non substitué ou substitué par un ou plusieurs restes alkyle linéaires ou ramifiés en $C_1$-$C_8$,

X représente soit un atome d'oxygène, soit un radical $-(CR_9R_{10})_n$ dans lequel n est égal à 1 ou 2 et $R_9$ et $R_{10}$ représentent un atome d'hydrogène ou un reste méthyle; dans le cas où X représente un radical $-(CR_9R_{10})_n$, les substituants $R_6$ et $R_8$ forment avec les atomes du cycle auxquels ils sont rattachés un système bicyclique contenant 7 ou 8 atomes de carbone; de plus, lorsque X représente un radical $-(CR_9R_{10})_n$- dans lequel n est égal à 1, $R_9$ et $R_{10}$ représentant un reste méthyle, $R_5$ représentant un atome d'hydrogène et $R_6$ et $R_6$ formant avec les atomes du cycle auxquels ils sont rattachés un système bicyclique contenant 7 atomes de carbone, $R_7$ peut également représenter un reste $-CH_2-SO_3H$, éventuellement sous forme de sel de métal alcalin, alcalino-terreux ou d'amine, sous réserve que lorsque $R_5$, $R_6$, $R_7$ et $R_8$ désignent un radical méthyle, X un atome d'oxygène, $R_2$ un atome d'hydrogène et $R_4$ un radical hydroxyle, l'un au moins des radicaux $R_1$ et $R_3$ désigne un radical hydroxyle, alkyle ou alcoxy.

2. Composé selon la revendication 1, caractérisé par le fait qu'il est choisi parmi la 3',4',5'-trihydroxy-4-benzyl-tétrahydro-2,2,5,5-tétraméthylfurane-3-one, la 3',5'-ditert.-butyl-4'-hydroxy-4-benzyl-tétrahydro-2,2,5,5-tétraméthyl-furane-3-one, la 3',5'-diisopropyl-4'-hydroxy-4-benzyl-tétrahydro-2,2,5,5-tétraméthylfurane-3-one, le 3',5'-ditert.-butyl-4'-hydroxy-3-benzyl-camphre, le 3',5'-diméthoxy-4'-hydroxy-3-benzyl-camphre, le 3',5'-diméthyl-4'-hydroxy-3-benzyl-camphre, le 3',5'-ditert.-butyl-4'-hydroxy-3-benzyl-nor-camphre, le 3',4',5'-trihydroxy-3-benzyl-camphre, le 3',5'-diisopropyl-4'-hydroxy-3-benzyl-camphre et le 3'-méthyl-4'-hydroxy-5'-tert.-butyl-3-benzyl-camphre.

3. Procédé de préparation du composé de formule (I') selon la revendication 1 ou 2, caractérisé par le fait qu'il consiste dans une première étape à condenser un aldéhyde aromatique de formule :

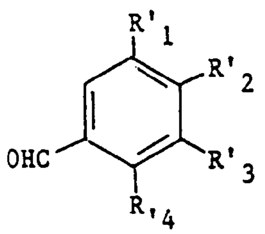

(III)

dans laquelle R'$_1$, R'$_2$, R'$_3$ et R'$_4$ ont les significations indiquées pour $R_1$ à $R_4$ dans la revendication 1, ou désignent un reste arylalcoxy,
sur une cyclanone de formule :

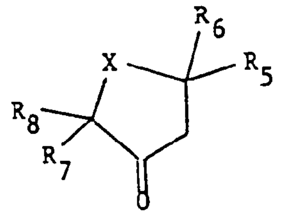

(IV)

dans laquelle $R_5$ à $R_8$ et X ont les significations indiquées dans la revendication 1, en présence d'un alcoolate de métal alcalin, dans un solvant, à une température comprise entre -78°C et le point d'ébullition du solvant, ou en présence d'une base minérale, dans un solvant, à une température comprise entre 0°C et le point d'ébullition du mélange réactionnel, ou en présence d'un borane de formule :

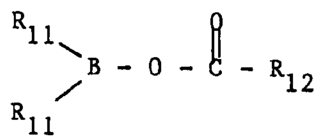

(V)

dans laquelle $R_{11}$ et $R_{12}$ représentent un reste alkyle en $C_1$-$C_6$, à une température de 150°C environ, sans solvant,
pour obtenir une benzylidène cyclanone de formule (II)

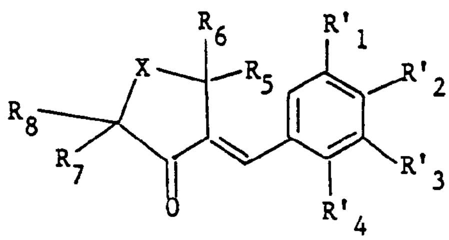

(II)

dans laquelle $R'_1$ à $R'_4$, $R_5$ à $R_5$ et X ont les significations indiquées ci-dessus, qu'on soumet dans une seconde étape à une réduction par hydrogénation catalytique ou par hydrogénation en présence d'un agent de transfert d'hydrogène et d'un catalyseur, en présence ou non d'un solvant inerte, à une température comprise entre -78°C et le point d'ébullition du mélange réactionnel.

**4.** Procédé de préparation du composé de formule (I') selon la revendication 1 ou 2, caractérisé par le fait qu'il consiste à condenser une base de Mannich de formule (VI) suivante :

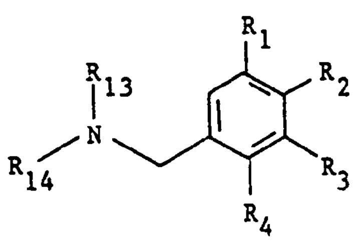

(VI)

formule dans laquelle $R_1$ à $R_4$ ont les significations mentionnées dans la revendication 1 et $R_{13}$ et $R_{14}$ désignent un reste alkyle en $C_1$-$C_4$ ou bien $R_{13}$ et $R_{14}$ forment, avec l'atome d'azote auxquels ils sont rattachés, un hétérocycle contenant 5 ou 6 atomes,
sur une cyclanone de formule (IV)

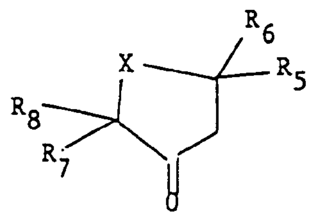

(IV)

dans laquelle $R_5$ à $R_8$ et X ont les significations indiquées dans la revendication 1, en présence d'un alcoolate de métal alcalin, dans un solvant, à une température comprise entre -78°C et le point d'ébullition du solvant.

**5.** Composition cosmétique, caractérisée par le fait qu'elle comprend, dans un support cosmétiquement acceptable, une quantité efficace d'au moins un composé de formule :

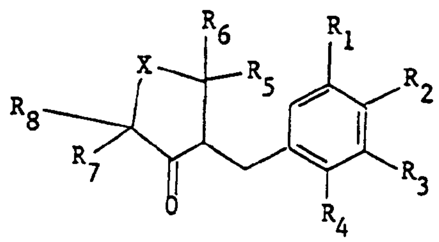

(I)

dans laquelle :

$R_1$ et $R_3$, identiques ou différents, représentent un atome d'hydrogène, un radical hydroxyle, un reste alkyle linéaire ou ramifié en $C_1$-$C_8$ ou un reste alcoxy linéaire ou ramifié en $C_1$-$C_8$,

$R_2$ et $R_4$, identiques ou différents, représentent un atome d'hydrogène ou un radical hydroxyle, étant entendu que l'un au moins des radicaux $R_2$ et $R_4$ représente un radical hydroxyle,

$R_5$, $R_6$, $R_7$ et $R_8$, identiques ou différents, représentent un atome d'hydrogène, un reste alkyle linéaire ou ramifié en $C_1$-$C_{18}$, un reste aralkyle tel que benzyle non substitué ou substitué par un reste alkyle en $C_1$-$C_4$ ou alcoxy en $C_1$-$C_4$, un reste aryle tel que phényle non substitué ou substitué par un reste alkyle en $C_1$-$C_4$; les substituants $R_5$ et $R_5$ et/ou les substituants $R_7$ et $R_5$ peuvent former, avec l'atome de carbone du cycle auquel ils sont rattachés, un cycle saturé contenant de 5 à 12 atomes de carbone non substitué ou substitué par un ou plusieurs restes alkyle linéaires ou ramifiés en $C_1$-$C_8$,

X représente soit un atome d'oxygène, soit un radical $-(CR_9R_{10})_n$ dans lequel n est égal à 1 ou 2 et $R_9$ et $R_{10}$ représentent un atome d'hydrogène ou un reste méthyle; dans le cas où X représente un radical $-(CR_9R_{10})_n$, les substituants $R_6$ et $R_8$ forment avec les atomes du cycle auxquels ils sont rattachés un système bicyclique contenant 7 ou 8 atomes de carbone; de plus, lorsque X représente un radical $-(CR_9R_{10})_n-$ dans lequel n est égal à 1, $R_9$ et $R_{10}$ représentant un reste méthyle, $R_5$ représentant un atome d'hydrogène et $R_5$ et $R_5$ formant avec les atomes du cycle auxquels ils sont rattachés un système bicyclique contenant 7 atomes de carbone, $R_7$ peut également représenter un reste $-CH_2-SO_3H$, éventuellement sous forme de sel de métal alcalin, alcalino-terreux ou d'amine.

**6.** Composition cosmétique selon la revendication 5, caractérisée par le fait qu'elle comprend à titre de composé (I) au moins l'un des composés choisis parmi : la 3',4',5'-trihydroxy-4-benzyl-tétrahydro-2,2,5,5-tétraméthylfurane-3-one, la 3',5'-ditert.-butyl-4'-hydroxy-4-benzyl-tétrahydro-2,2,5,5-tétraméthyl-furane-3-one, la 3',5'-diisopropyl-4'-hydroxy-4-benzyl-tétrahydro-2,2,5,5-tétraméthyl-furane-3-one, le 3',5'-ditert.-butyl-4'-hydroxy-3-benzyl-camphre, le 3',5'-diméthoxy-4'-hydroxy-3-benzyl-camphre, le 3',5'-diméthyl-4'-hydroxy-3-benzyl-camphre, le 3',5'-ditert.-butyl-4'-hydroxy-3-benzyl-nor-camphre, le 3',4',5'-trihydroxy-3-benzyl-camphre, le 3',5'-diisopropyl-4'-hydroxy-3-benzyl-camphre et le 3'-méthyl-4'-hydroxy-5'-tert.-butyl-3-benzyl-camphre.

**7.** Composition cosmétique selon la revendication 5 ou 6, caractérisée par le fait qu'elle se présente sous forme de lotion, émulsion, gel oléoalcoolique ou hydroalcoolique, bâtonnet solide ou aérosol.

**8.** Composition cosmétique selon la revendication 7, caractérisée par le fait qu'elle contient en outre des adjuvants cosmétiques choisis parmi les épaississants, adoucissants, humectants, tensioactifs, conservateurs, anti-mousses, parfums, huiles, cires, lanoline, monoalcools et polyols inférieurs, propulseurs, colorants et pigments.

9. Composition cosmétique selon la revendication 7 ou 8, caractérisée par le fait qu'elle constitue une composition protectrice de l'épiderme humain et contient 0,1 à 8% en poids de composé de formule (I).

10. Composition cosmétique selon la revendication 7 ou 8, se présentant sous forme de composition antisolaire, caractérisée par le fait qu'elle contient 0,1 à 8% en poids de composé de formule (I) et 0,5 à 15% en poids de filtres UV-B et UV-A.

11. Composition cosmétique selon la revendication 5 ou 6, se présentant sous forme d'une composition cosmétique colorée ou non, stabilisée à l'oxydation, caractérisée par le fait qu'elle est constituée par une composition capillaire, un produit de maquillage ou une composition pour les soins ou le traitement de la peau, comprenant 0,1 à 8% en poids de composé de formule (I).

12. Composition cosmétique selon la revendication 5 ou 6, destinée à protéger les cheveux, caractérisée par le fait qu'elle se présente sous forme de shampooing, de lotion, gel ou émulsion à rincer, de lotion ou gel coiffants, ou traitants, de lotion ou gel pour le brushing ou la mise en plis, de laque pour cheveux, de composition de permanente, de coloration ou de décoloration et qu'elle contient 0,25 à 8% en poids de composé de formule (I).

13. Procédé de protection d'une composition cosmétique contre l'oxydation, caractérisé par le fait qu'il consiste à incorporer à cette composition une quantité efficace d'au moins un composé de formule (I) selon la revendication 5 ou 6.

14. Utilisation d'au moins un composé de formule (I) selon la revendication 5 ou 6 en tant qu'agent antioxydant.

15. Utilisation d'au moins un composé de formule (I) selon la revendication 5 ou 6 à titre de produit cosmétique.

16. Composé de formule (I) selon la revendication 5 ou 6 pour son utilisation comme médicament.

17. Composé de formule (I) selon la revendication 5 ou 6 pour son utilisation dans le traitement des inflammations et allergies cutanées.

18. Composition pharmaceutique, caractérisée par le fait qu'elle comprend une quantité efficace d'au moins un composé de formule (I) selon la revendication 5 ou 6, dans un support ou excipient non toxique.

19. Composition pharmaceutique destinée à être administrée par voie topique, se présentant sous forme de crème, onguent, pommade, solution, gel, lotion, spray, suspension, caractérisée par le fait qu'elle contient au moins un composé de formule (I) selon la revendication 5 ou 6.

20. Composition pharmaceutique destinée à être administrée par voie orale sous forme de comprimés, de gélules, de dragées, de sirops, de suspensions, de solutions, d'émulsions, caractérisée par le fait qu'elle contient au moins un composé de formule (I) selon la revendication 5 ou 6.

21. Composition pharmaceutique selon la revendication 20, caractérisée par le fait qu'elle contient le composé actif de formule (I) dans des proportions comprises entre 0,25 et 8% en poids par rapport au poids total de la composition.

22. Utilisation d'un composé de formule (I) selon la revendication 5 ou 6 pour la préparation d'un médicament destiné au traitement des inflammations et allergies cutanées.

## Patentansprüche

1. Benzylcyclanonderivat gemäß der Formel:

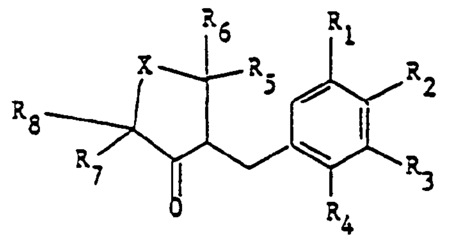

(I')

worin:

$R_1$ und $R_3$, gleich oder verschieden, ein Wasserstoffatom, einen Hydroxylrest, einen linearen oder verzweigten $C_{1-8}$-Alkyl- oder -Alkoxyrest darstellen,

$R_3$ und $R_4$, gleich oder verschieden, ein Wasserstoffatom oder einen Hydroxylrest darstellen, wobei mindestens einer der Reste $R_2$ und $R_4$ einen Hydroxylrest darstellt,

$R_5$, $R_6$, $R_7$ und $R_8$, gleich oder verschieden, ein Wasserstoffatom, einen linearen oder verzweigten $C_{1-18}$-Alkylrest, einen Aralkylrest wie einen nicht-substituierten oder durch einen $C_{1-4}$-Alkyl- oder -Alkoxyrest substituierten Benzylrest, einen Arylrest wie einen nicht-substituierten oder durch einen $C_{1-4}$-Alkylrest substituierten Phenylrest darstellen; die Substituenten $R_5$ und $R_6$ und/oder die Substituenten $R_7$ und $R_8$ mit dem Kohlenstoffatom des Ringes, an den sie gebunden sind, einen gesättigten Ring mit 5 bis 12 Kohlenstoffatomen bilden können, der nicht substituiert oder durch einen oder mehrere lineare oder verzweigte $C_{1-8}$-Alkylreste substituiert ist,

X entweder ein Sauerstoffatom oder einen Rest $-(CR_9R_{10})_n$ darstellt, worin n gleich 1 oder 2 ist und $R_9$ und $R_{10}$ ein Wasserstoffatom oder einen Methylrest darstellen; und wenn X einen Rest $(CR_9R_{10})_n$ darstellt, die Substituenten $R_8$ und $R_8$ mit den Atomen des Ringes, an die sie gebunden sind, ein bizyklisches System mit 7 oder 8 Kohlenstoffatomen bilden; und ausserdem, wenn X einen Rest $-(CR_9R_{10})_n-$ darstellt, worin n gleich 1 ist, wobei $R_9$ und $R_{10}$ einen Methylrest und $R_5$ ein Wasserstoffatom darstellen und $R_6$ und $R_8$ mit den Ringatomen, an die sie gebunden sind, ein bizyklisches System mit 7 Kohlenstoffatomen bilden, $R_7$ auch einen Rest $-CH_2-SO_3H$ darstellen kann, der gegebenenfalls in Form eines Alkali-, Erdalkalimetall- oder Aminsalzes vorliegt,mit der Maßgabe, daß, wenn $R_5$, $R_6$, $R_7$, und $R_8$ einen Methylrest, X ein Sauerstoffatom, $R_2$ ein Wasserstoffatom und $R_4$ einen Hydroxylrest bedeuten, mindestens einer der Reste $R_1$ und $R_3$ einen Hydroxyl-, Alkyl- oder Alkoxyrest bedeutet.

**2.** Verbindung gemäß Anspruch 1, dadurch **gekennzeichnet**, daß sie ausgewählt ist aus
- 3',4',5'-Trihydroxy-4-benzyltetrahydro-2,2,5,5-tetramethylfuran-3-on,
- 3',5'-Di-t-butyl-4'-hydroxy-4-benzyltetrahydro-2,2,5,5-tetramethylfuran-3-on,
- 3',5'-Diisopropyl-4'-hydroxy-4-benzyltetrahydro-2,2,5,5-tetramethylfuran-3-on,
- 3',5'-Di-t-butyl-4'-hydroxy-3-benzylkampfer,
- 3',5'-Dimethoxy-4'-hydroxy-3-benzylkampfer,
- 3',5'-Dimethyl-4'-hydroxy-3-benzylkampfer,
- 3',5'-Di-t-butyl-4'-hydroxy-3-benzylnorkampfer,
- 3',4',5'-Trihydroxy-3-benzylkampfer,
- 3',5'-Diisopropyl-4'-hydroxy-3-benzylkampfer
- 3'-Methyl-4'-hydroxy-5'-t-butyl-3-benzylkampfer.

**3.** Verfahren zur Herstellung einer Verbindung der Formel (I') gemäß Anspruch 1 oder 2, dadurch **gekennzeichnet**, daß man in einer ersten Stufe einen aromatischen Aldehyd der Formel:

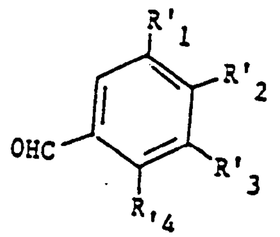

(III)

worin $R'_1$, $R'_2$, $R'_3$ und $R'_4$ die für $R_1$ bis $R_4$ in Anspruch 1 angegebenen Bedeutungen haben oder einen Arylalkoxyrest bedeuten,
mit einem Cyclanon der Formel:

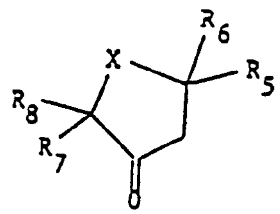

(IV)

worin $R_5$ bis $R_8$ und X die in Anspruch 1 angegebenen Bedeutungen haben, in Gegenwart eines Alkalimetallalkoholats in einem Lösungsmittel bei einer Temperatur von -78°C bis zur Siedepunkt des Lösungsmittels oder in Gegenwart einer Mineralbase in einem Lösungsmittel bei einer Temperatur von 0°C bis zum Siedepunkt der Reaktionsmischung oder in Gegenwart eines Borans der Formel:

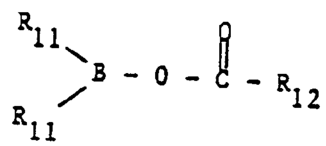

(V)

worin $R_{11}$ und $R_{12}$ einen $C_{1-6}$-Alkylrest darstellen, bei einer Temperatur von ca. 150°C ohne Lösungsmittel, kondensiert,
um ein Benzylidencyclanon der Formel (II):

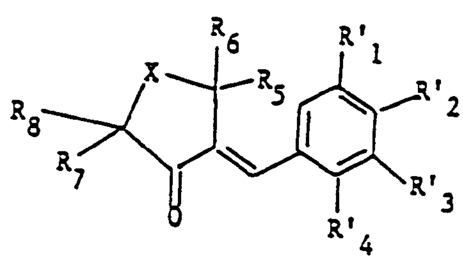

(II)

worin $R'_1$ bis $R'_4$, $R_5$ bis $R_5$ und X die oben angegebenen Bedeutungen haben, zu erhalten, welches man in einer zweiten Stufe durch katalytische Hydrierung oder durch Hydrierung in Gegenwart eines Wasserstoffübertragungsmittels und eines Katalysators, in Gegenwart eines inerten Lösungsmittels oder nicht, bei einer Temperatur von -78°C bis zum Siedepunkt der Reaktionsmischung reduziert.

4. Verfahren zur Herstellung der Verbindung der Formel (I') gemäß Anspruch 1 oder 2, dadurch **gekennzeichnet**, daß
man eine Mannich-Base der folgenden Formel (VI):

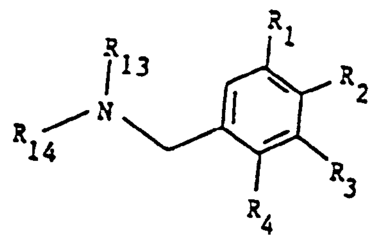

(VI)

worin $R_1$ bis $R_4$ die in Anspruch 1 angegebenen Bedeutungen haben und $R_{13}$ und $R_{14}$ einen $C_{1-4}$-Alkylrest bedeuten, oder $R_{13}$ und $R_{14}$ mit dem Stickstoffatom, an das sie gebunden sind, auch einen Heterozyklus mit 5 oder 6 Atomen bilden,
mit einem Cyclanon der Formel (IV):

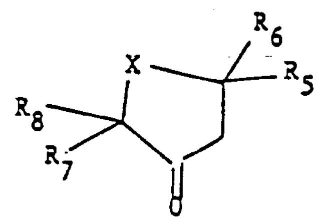

(IV)

worin $R_5$ bis $R_8$ und X die in Anspruch 1 angegebenen Bedeutungen haben, in Gegenwart eines Alkalimetallalkoholats in einem Lösungsmittel bei einer Tempratur von -78°C bis zum Siedepunkt des Lösungsmittels kondensiert.

**5.** Kosmetische Zusammensetzung,
dadurch **gekennzeichnet**, daß
sie in einem kosmetisch verträglichen Träger eine wirksame Menge mindestens einer Verbindung der Formel enthält:

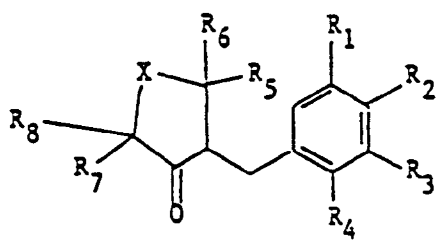

(I)

worin:

$R_1$ und $R_3$, gleich oder verschieden, ein Wasserstoffatom, einen Hydroxylrest, einen linearen oder verzweigten $C_{1-8}$-Alkyl- oder
-Alkoxyrest darstellen,

$R_3$ und $R_4$, gleich oder verschieden, ein Wasserstoffatom oder einen Hydroxylrest darstellen, wobei mindestens einer der Reste $R_2$ und $R_4$ einen Hydroxylrest darstellt,

$R_5$, $R_6$, $R_7$ und $R_5$, gleich oder verschieden, ein Wasserstoffatom, einen linearen oder verzweigten $C_{1-18}$-Alkylrest, einen Aralkylrest wie einen nicht-substituierten oder durch einen $C_{1-4}$-Alkyl- oder -Alkoxyrest substituierten Benzylrest, einen Arylrest wie einen nicht-substituierten oder durch einen $C_{1-4}$-Alkylrest substituierten Phenylrest darstellen; die Substituenten $R_5$ und $R_6$ und/oder die Substituenten $R_7$ und $R_5$ mit dem Kohlenstoffatom des Ringes, an den sie gebunden sind, einen gesättigten Ring mit 5 bis 12 Kohlenstoffatomen bilden können, der nicht substituiert oder durch einen oder mehrere lineare oder verzweigte $C_{1-8}$-Alkylreste substituiert ist,

X entweder ein Sauerstoffatom oder einen Rest $-(CR_9R_{10})_n$ darstellt, worin n gleich 1 oder 2 ist und $R_9$ und $R_{10}$ ein Wasserstoffatom oder ein Methylrest darstellen; und wenn X einen Rest $(CR_9R_{10})_n$ darstellt, die Substituenten $R_6$ und $R_8$ mit den Atomen des Ringes, an die sie gebunden sind, ein bizyklisches System mit 7 oder 8 Kohlenstoffatomen bilden; und ausserdem, wenn X einen Rest $-(CR_9R_{10})_n-$ darstellt,

worin n gleich 1 ist, wobei $R_9$ und $R_{10}$ einen Methylrest und $R_5$ ein Wasserstoffatom darstellen und $R_6$ und $R_8$ mit den Ringatomen, an die sie gebunden sind, ein bizyklisches System mit 7 Kohlenstoffatomen bilden, $R_7$ auch einen Rest $-CH_2-SO_3H$ darstellen kann, der gegebenenfalls in Form eines Alkali-, Erdalkalimetall- oder Aminsalzes vorliegt.

**6.** Kosmetiche Zusammensetzung nach Anspruch 5,
dadurch **gekennzeichnet**, daß
sie als Verbindung (I) mindestens eine der Verbindungen enthält, ausgewählt aus:
- 3',4',5'-Trihydroxy-4-benzyltetrahydro-2,2,5,5-tetramethylfuran-3-on,
- 3',5'-Di-t-butyl-4'-hydroxy-4-benzyltetrahydro-2,2,5,5-tetramethylfuran-3-on,
- 3',5'-Diisopropyl-4'-hydroxy-4-benzyltetrahydro-2,2,5,5-tetramethylfuran-3-on,
- 3',5'-Di-t-butyl-4'-hydroxy-3-benzylkampfer,
- 3',5'-Dimethoxy-4'-hydroxy-3-benzylkampfer,
- 3',5'-Dimethyl-4'-hydroxy-3-benzylkampfer,
- 3',5'-Di-t-butyl-4'-hydroxy-3-benzylnorkampfer,
- 3',4',5'-Trihydroxy-3-benzylkampfer,
- 3',5'-Diisopropyl-4'-hydroxy-3-benzylkampfer,
- 3'-Methyl-4'-hydroxy-5'-t-butyl-3-benzylkampfer.

**7.** Kosmetische Zusammensetzung gemäß Anspruch 5 oder 6,
dadurch **gekennzeichnet**, daß
sie in Form einer Lotion, Emulsion, eines oleoalkoholischen oder hydroakoholischen Gels, eines Feststoffstäbchens oder Aerosols vorliegt.

**8.** Kosmetische Zusammensetzung gemäß Anspruch 7,
dadurch **gekennzeichnet**, daß
sie ausserdem kosmetische Hilfstoffe enthält, ausgewählt aus Verdickungsmitteln, Weichmachern, Feuchtigkeitsmitteln, oberflächenaktiven Mitteln, Konservierungsmitteln, Antischaummitteln, Parfüm, Ölen, Wachsen, Lanolin, Niedrigmonoalkoholen und -polyolen, Treibmitteln, Färbemitteln und Pigmenten.

**9.** Kosmetische Zusammensetzung gemäß Anspruch 7 oder 8,
dadurch **gekennzeichnet**, daß
sie eine Zusammensetzung zum Schutz der menschlichen Haut darstellt und 0,1 bis 8 Gew.% der Verbindung der Formel (I) enthält.

**10.** Kosmetische Zusammensetzung gemäß Anspruch 7 oder 8, die in Form einer Zusammensetzung zum Schutz vor schädlicher Sonnenstrahleinwirkung vorliegt,
dadurch **gekennzeichnet**, daß
sie 0,1 bis 8 Gew% Verbindung der Formel (I) und 0,5 bis 15 Gew.% Filtermittelstoffe UV-B und UV-A enthält.

**11.** Kosmetische Zusammensetzung gemäß Anspruch 5 oder 6, die in Form einer gefärbten oder nicht gefärbten kosmetischen Zusammensetzung vorliegt und gegenüber Oxidation stabilisiert ist,
dadurch **gekennzeichnet**, daß
sie eine Zusammensetzung für das Haar, ein Schminkprodukt oder eine Zusammensetzung zur Pflege oder Behandlung der Haut darstellt, enthaltend 0,1 bis 8 Gew.% der Verbindung der Formel (I).

**12.** Kosmetische Zusammensetzung gemäß Anspruch 5 oder 6 zum Schutz der Haare, dadurch **gekennzeichnet**, daß sie in Form eines Schampoos, einer Lotion, eines Gels oder einer Emulsion zur Spülung, einer Frisur- oder Behandlungslotion oder eines entsprechenden Gels, einer Lotion oder eines Gels zum Bürsten oder zur Wellengebung, eines Haarlackes, einer Zusammensetzung zur Dauerwelle, Färbung oder Entfärbung vorliegt und 0,25 bis 8 Gew.% der Verbindung der Formel (I) enthält.

**13.** Verfahren zum Schutz einer kosmetischen Zusammensetzung vor Oxidation, dadurch **gekennzeichnet**, daß man dieser Zusammensetzung eine wirksame Menge mindestens einer Verbindung der Formel (I) gemäß Anspruch 5 oder 6 einverleibt.

**14.** Verwendung mindestens einer Verbindung der Formel (I) gemäß Anspruch 5 oder 6 als Antioxidans.

**15.** Verwendung mindestens einer Verbindung der Formel (I) gemäß Anspruch 5 oder 6 als Kosmetikprodukt.

**16.** Verbindung der Formel (I) gemäß Anspruch 5 oder 6 zur Verwendung als Medikament.

**17.** Verbindung der Formel (I) gemäß Anspruch 5 oder 6 zur Verwendung bei der Behandlung von Hautentzündungen und - allergien.

**18.** Pharmazeutische Zusammensetzung,
dadurch **gekennzeichnet**, daß
sie eine wirksame Menge mindestens einer Verbindung der Formel (I) gemäß Anspruch 5 oder 6 in einem nicht toxischen Träger oder Exzipienten enthält.

**19.** Pharmazeutische Zusammensetzung zur Verabreichung auf topischem Weg, die in Form einer Creme, Salbe, Pomade, Lösung, eines Gels, einer Lotion, eines Sprays, einer Suspension vorliegt,
dadurch **gekennzeichnet**, daß
sie mindestens eine Verbindung der Formel (I) gemäß Anspruch 5 oder 6 enthält.

**20.** Pharmazeutische Zusammensetzung zur Verabreichung auf oralem Weg in Form von Tabletten, Kapseln, Dragees, Sirups, Suspensionen, Lösungen, Emulsionen, dadurch **gekennzeichnet**, daß
sie mindestens eine Verbindung der Formel (I) gemäß Anspruch 5 oder 6 enthält.

**21.** Pharmazeutische Zusammensetzung gemäß Anspruch 20,
dadurch **gekennzeichnet,** daß
sie die Wirkverbindung der Formel (I) in Mengenverhältnissen von 0,25 bis 8 Gew.%, bezogen auf Gesamtgewicht der Zusammensetzung, enthält.

**22.** Verwendung einer Verbindung der Formel (I) gemäß Anspruch 5 oder 6 zur Herstellung eines Medikaments zur Behandlung von Hautentzündungen und -allergien.

## Claims

**1.** Benzylcyclanone derivative, characterized in that it corresponds to the formula:

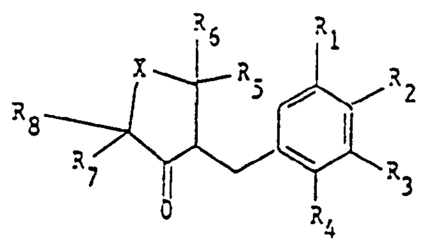

(I')

in which:

$R_1$ and $R_3$, which may be identical or different, represent a hydrogen atom, a hydroxyl radical, a linear or branched $C_1$-$C_8$ alkyl residue or a linear or branched $C_1$-$C_8$ alkoxy residue,

$R_2$ and $R_4$, which may be identical or different, represent a hydrogen atom or a hydroxyl radical, on the understanding that at least one of the radicals $R_2$ and $R_4$ represents a hydroxyl radical,

$R_5$, $R_6$, $R_7$ and $R_8$, which may be identical or different, represent a hydrogen atom, a linear or branched $C_1$-$C_{18}$ alkyl residue, an aralkyl residue such as benzyl, unsubstituted or substituted with a $C_1$-$C_4$ alkyl or $C_1$-$C_4$ alkoxy residue, or an aryl residue such as phenyl, unsubstituted or substituted with a $C_1$-$C_4$ alkyl residue; the substituents $R_6$ and $R_6$ and/or the substituents $R_7$ and $R_8$, with the carbon atom of the ring to which they are attached, can form a saturated ring containing from 5 to 12 carbon atoms, unsubstituted or substituted with one or more linear or branched $C_1$-$C_8$ alkyl residues,

X represents either an oxygen atom, or a radical $-(CR_9R_{10})_n$ in which n is equal to 1 or 2 and $R_9$ and $R_{10}$ represent a hydrogen atom or a methyl residue; in the case where X represents a radical $(CR_9R_{10})_n$, the substituents $R_6$ and $R_8$, with the atoms of the ring to which they are attached, form a bicyclic system containing 7 or 8 carbon atoms; furthermore, when X represents a radical $-(CR_9R_{10})_n-$ in which n is equal to 1, $R_9$ and $R_{10}$ represent a methyl residue, $R_6$ representing a hydrogen atom and $R_6$ and $R_8$,

with the atoms of the ring to which they are attached, forming a bicyclic system containing 7 carbon atoms, $R_7$ can also represent a $-CH_2-SO_3H$ residue, optionally in the form of an alkali metal, alkaline earth metal or amine salt, with the proviso that, when $R_5$, $R_6$, $R_7$ and $R_8$ denote a methyl radical, X an oxygen atom, $R_2$ a hydrogen atom and $R_4$ a hydroxyl radical, at least one of the radicals $R_1$ and $R_3$ denotes a hydroxyl, alkyl or alkoxy radical.

2. Compound according to Claim 1, characterized in that it is selected from 3',4',5'-trihydroxy-4-benzyltetrahydro-2,2,5,5-tetramethyl-3-furanone, 3'5'-di-tert-butyl-4'-hydroxy-4-benzyltetrahydro-2,2,5,5-tetramethyl-3-furanone, 3',5'-diisopropyl-4'-hydroxy-4-benzyltetrahydro-2,2,5,5-tetramethyl-3-furanone, 3',5', -di-tertbutyl-4'-hydroxy-3-benzylcamphor, 3',5'-dimethoxy-4'-hydroxy-3-benzylcamphor, 3',5'-dimethyl-4'-hydroxy-3-benzylcamphor, 3',5'-di-tert-butyl-4'-hydroxy-3-benzylnorcamphor, 3',4',5'-trihydroxy-3-benzylcamphor, 3',5'-diisopropyl-4'-hydroxy-3-benzylcamphor and 3'-methyl-4'-hydroxy-5'-tert-butyl-3-benzylcamphor.

3. Process for preparing the compound of formula (I') according to Claim 1 or 2, characterized in that it consists, in a first step, in condensing an aromatic aldehyde of formula:

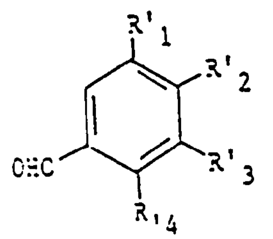

(III)

in which $R'_1$, $R'_2$ , $R'_3$ and $R'_4$ have the meanings stated for $R_1$ to $R_4$ in Claim 1, or denote an arylalkoxy residue, with a cyclanone of formula:

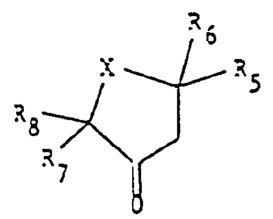

(IV)

in which $R_5$ to $R_8$ and X have the meanings as stated in Claim 1, in the presence of an alkali metal alcoholate, in a solvent, at a temperature between -78°C and the boiling point of the solvent, or in the presence of an inorganic base, in a solvent, at a temperature between 0°C and the boiling point of the reaction mixture, or in the presence of a borane of formula:

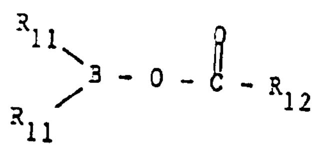

. (V)

in which $R_{11}$ and $R_{l2}$ represent a $C_1$-$C_6$ alkyl residue, at a temperature of approximately 150°C, without a solvent, to obtain a benzylidenecyclanone of formula (II)

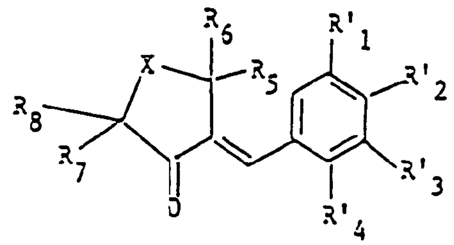

(II)

in which $R'_1$ to $R'_4$, $R_5$ to $R_8$ and X have the meanings as stated above, which is subjected in a second step to a reduction by catalytic hydrogenation, or by hydrogenation in the presence of a hydrogen transfer agent and a catalyst, in the presence or absence of an inert solvent, at a temperature between -78°C and the boiling point of the reaction mixture.

4. Process for preparing the compound of formula (I') according to Claim 1 or 2, characterized in that it consists in condensing a Mannich base of the following formula (VI):

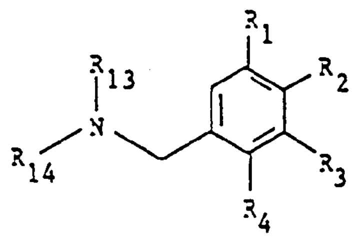

(VI)

in which formula $R_1$ to $R_4$ have the meanings stated in Claim 1 and $R_{13}$ and $R_{14}$ denote a $C_1$-$C_4$ alkyl residue, or alternatively $R_{13}$ and $R_{14}$, with the nitrogen atom to which they are attached, form a heterocycle containing 5 or 6 atoms,
with a cyclanone of formula (IV) :

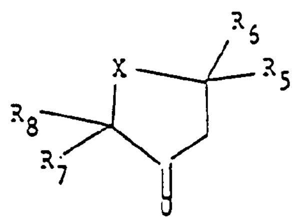

(IV)

in which $R_5$ to $R_8$ and X have the meanings as stated in Claim 1, in the presence of an alkali metal alcoholate, in a solvent, at a temperature between -78°C and the boiling point of the solvent.

5. Cosmetic composition, characterized in that it comprises, in a cosmetically acceptable vehicle, an effective amount of at least one compound of formula:

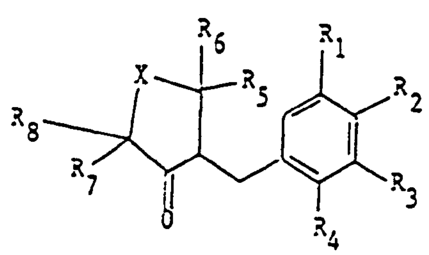

(I)

in which:

$R_1$ and $R_3$, which may be identical or different, represent a hydrogen atom, a hydroxyl radical, a linear or branched $C_1$-$C_8$ alkyl residue or a linear or branched $C_1$-$C_8$ alkoxy residue,

$R_2$ and $R_4$, which may be identical or different, represent a hydrogen atom or a hydroxyl radical, on the understanding that at least one of the radicals $R_2$ and $R_4$ represents a hydroxyl radical,

$R_5$, $R_6$ $R_7$ and $R_8$, which may be identical or different, represent a hydrogen atom, a linear or branched $C_1$-$C_{18}$ alkyl residue, an aralkyl residue such as benzyl, unsubstituted or substituted with a $C_1$-$C_4$ alkyl or $C_1$-$C_4$ alkoxy residue, or an aryl residue such as phenyl, unsubstituted or substituted with a $C_1$-$c_4$ alkyl residue; the substituents $R_5$ and $R_6$ and/or the substituents $R_7$ and $R_8$, with the carbon atom of the ring to which they are attached, can form a saturated ring containing from 5 to 12 carbon atoms, unsubstituted or substituted with one or more linear or branched $C_1$-$C_8$ alkyl residues,

X represents either an oxygen atom, or a radical $-(CR_9R_{10})_n$ in which n is equal to 1 or 2 and $R_9$ and $R_{10}$ represent a hydrogen atom or a methyl residue; in the case where X represents a radical $(CR_9R_{10})_n$, the substituents $R_5$ and $R_8$, with the atoms of the ring to which they are attached, form a bicyclic system containing 7 or 8 carbon atoms; furthermore, when X represents a radical $-(CR_9R_{10})_n-$ in which n is equal to 1, $R_9$ and $R_{10}$ represent a methyl residue, R5 representing a hydrogen atom and $R_6$ and $R_8$, with the atoms of the ring to which they are attached, forming a bicyclic system containing 7 carbon atoms, $R_7$ can also represent a $-CH_2-SO_3H$ residue, optionally in the form of an alkali metal, alkaline earth metal or amine salt.

**6.** Cosmetic composition according to Claim 5, characterized in that it comprises, by way of a compound (I), at least one of the compounds selected from: 3',4',5'-trihydroxy-4-benzyltetrahydro-2,2,5,5-tetramethyl-3-furanone, 3'5'-di-tert-butyl-4'-hydroxy-4-benzyltetrahydro-2,2,5,5-tétramethyl-3-furanone,3',5'-diisopropyl-4'-hydroxy-4-benzyltetrahydro-2,2,5,5-tetra-methyl-3-furanone, 3',5',-di-tert-butyl-4'-hydroxy-3-benzylcamphor, 3',5'-dimethoxy-4'-hydroxy-3-benzylcamphor, 3',5'-dimethyl-4'-hydroxy-3-benzylcamphor, 3',5'-di-tert-butyl-4'-hydroxy-3 benzylnorcamphor, 3',4',5'-trihydroxy-3-benzylcamphor, 3',5'-diisopropyl-4'-hydroxy-3-benzylcamphor and 3'-methyl-4'-hydroxy-5'-tert-butyl-3-benzylcamphor.

**7.** Cosmetic composition according to Claim 5 or 6, characterized in that it is presented in the form of a lotion, emulsion, oleoalcoholic or aqueous-alcoholic gel, solid stick or aerosol.

**8.** Cosmetic composition according to Claim 7, characterized in that it contains, in addition, cosmetic adjuvants selected from thickeners, emollients, humectants, surfactants, preservatives, antifoams, fragrances, oils, waxes, lanolin, lower monohydric alcohols and polyols, propellants, colourings and pigments.

**9.** Cosmetic composition according to Claim 7 or 8, characterized in that it constitutes a composition for protecting the human epidermis and contains 0.1 to 8% by weight of compound of formula (I).

**10.** Cosmetic composition according to Claim 7 or 8, presented in the form of an antisun composition, characterized in that it contains 0.1 to 8% by weight of compound of formula (I) and 0.5 to 15% by weight of UV-3 and UV-A screening agents.

**11.** Cosmetic composition according to Claim 5 or 6, presented in the form of a coloured or uncoloured cosmetic composition, stabilized to oxidation, characterized in that it consists of a hair-care composition, a makeup product or a skin-care or -treatment composition, comprising 0.1 to 8% by weight of compound of formula (I).

**12.** Cosmetic composition according to Claim 5 or 6, intended for protection of the hair, characterized in that it is presented in the form of a shampoo, a lotion, gel or emulsion to be rinsed, a styling or treatment lotion or gel, a blow-drying or setting lotion or gel, a hair lacquer or a permanent-waving, dyeing or bleaching composition, and in that it contains 0.25 to 8% by weight of compound of formula (I).

**13.** Process for protecting a cosmetic composition against oxidation, characterized in that it consists in incorporating an effective amount of at least one compound of formula (I) according to Claim 5 or 6 in this composition.

**14.** Use of at least one compound of formula (I) according to Claim 5 or 6 as an antioxidant agent.

**15.** Use of at least one compound of formula (I) according to Claims 5 or 6 by way of a cosmetic product.

**16.** Compound of formula (I) according to Claim 5 or 6, for its use as a medicinal product.

**17.** Compound of formula (I) according to Claim 5 or 6, for its use in the treatment of skin allergies and inflammations.

**18.** Pharmaceutical composition, characterized in that it comprises an effective amount of at least one compound of formula (I) according to Claim 5 or 6, in a non-toxic vehicle or excipient.

**19.** Pharmaceutical composition intended for topical administration, presented in the form of a cream, ointment, pomade, solution, gel, lotion, spray or suspension, characterized in that it contains at least one compound of formula (I) according to Claim 5 or 6.

**20.** Pharmaceutical composition intended for oral administration in the form of tablets, hard gelatin capsules, dragées, syrups, suspensions, solutions or emulsions, characterized in that it contains at least one compound of formula (I) according to Claim 5 or 6.

**21.** Pharmaceutical composition according to Claim 20, characterized in that it contains the active compound of formula (I) in proportions of between 0.25 and 8% by weight relative to the total weight of the composition.

**22.** Use of a compound of formula (I) according to Claim 5 or 6 for the preparation of a medicinal product intended for the treatment of skin allergies and inflammations.